# EUROPÄISCHE PATENTSCHRIFT

(11) **EP 2 886 542 B1**
(45) Veröffentlichungstag und Bekanntmachung des Hinweises auf die Patenterteilung: **18.04.2018**
(21) Anmeldenummer: 14200043.9
(22) Anmeldetag: 23.12.2014
(51) Int. Cl.: C07D 413/06, C07D 413/14, C07D 413/04, C09B 62/00, G01N 33/533, C09B 57/00, C09B 69/00

(54) **Markerfarbstoffe für UV- und kurzwellige Anregung mit hohem Stokes Shift auf der Basis von Benzoxazolen**
Marker dyes for UV and short-wave excitation with high Stokes shift on the basis of benzoxazoles
Colorants de marqueur à base de benzoxazoles pour l'excitation UV et à ondes courtes avec grand décalage de Stokes

(30) Priorität: 23.12.2013 DE 102013114848
(43) Veröffentlichungstag der Anmeldung: 24.06.2015
(73) Patentinhaber: Dyomics GmbH, 07745 Jena (DE)
(72) Erfinder: Schweder, Bernd G., 07745 Jena (DE); Wenzel, Matthias S., 07749 Jena (DE); Frank, Wilhelm G., 07743 Jena (DE); Lehmann, Frank G., 07749 Jena (DE); Czerney, Peter T., 99425 Weimar (DE)
(74) Vertreter: Oehmke, Volker

(56) Entgegenhaltungen:
- WO-A1-2007/038659
- KUBIN R F ET AL: "Anionic and zwitterionic photophysical effects in some pyridinium oxazole laser dyes", LASER CHEMISTRY, HARWOOD ACADEMIC PUBLISHERS, CHUR, CH, Bd. 10, Nr. 4, 1. Januar 1990 (1990-01-01) , Seiten 247-258, XP009130559, ISSN: 0278-6273, DOI: 10.1155/1990/12860
- FLETCHER A N ET AL: "Fluorescence and lasing characteristics of some long-lived flashlamp-pumpable, oxazole dyes", OPTICS COMMUNICATIONS, NORTH-HOLLAND PUBLISHING CO. AMSTERDAM, NL, Bd. 48, Nr. 5, 1. Januar 1984 (1984-01-01) , Seiten 352-356, XP024477882, ISSN: 0030-4018, DOI: 10.1016/0030-4018(84)90314-6 [gefunden am 1984-01-01]

## Beschreibung

Fluoreszenzbasierte Marker finden seit Jahrzehnten Einsatz in der biologischen, biotechnologischen und medizinischen Forschung sowie in der medizinischen Diagnostik [Brinkley M., A Brief Survey of Methods for Preparing Protein Conjugates with Dyes, Haptens and Cross-Linking Reagents, Bioconjugate Chem, 3 (1992) 2-12; Waggoner A., Covalent Labeling of Proteins and Nucleic Acids with Fluorophores, Meth. Enzymol., 246 (1995) 362-373]. Ursprünglich wurden aus dem Bereich der Textil- und Sensibilisatorfarbstoffe bekannte Chromophore derivatisiert, um sie für Anwendungen im wässrig-physiologischen Milieu zu optimieren. Besondere Schwerpunkte der Entwicklungen stellten hier die Wasserlöslichkeit sowie eine hohe Quantenausbeute in wässriger Lösung dar, wobei Ausgangspunkt solcher Entwicklungen meist Filmfarbstoffe waren [Poppe E.-J., Sensibilisierungsfarbstoffe mit sauren N-Substituenten, Z. wiss. Photographie, 63 (1969) 149-158 und darin zitierte Arbeiten].

Häufig verwendete Farbstoff-Klassen stellen hier die Xanthylium- (Fluorescein, Rhodole, Rhodamine), Cyanin- (Carbo-, Di-Carbo- und Tri-Carbo-), Dipyrrhomethen- (BoDiPy-) und Cumarin-Chromophore dar, wobei die Vielfalt der Farbstoffe nicht auf die genannten Typen beschränkt ist [Panchuk-Voloshina N. et al., Alexa Dyes, a Series of New Fluorescent Dyes that Yield Exceptionally Bright, Photostable Conjugates, J. Histochem. Cytochem., 47 (1999) 1179-1188].

Ausgehend von kommerziell verfügbaren Lichtquellen wie Quecksilber- und Xenon-Brennern sowie Wolfram-Lampen und bedingt durch die spektrale Beschränkung des für das menschliche Auge sichtbaren Lichts erfolgten die Fluoreszenzmarker-Entwicklungen zunächst für zwischen 400 nm und ca. 650 nm emittierende Chromophore.

Das Aufkommen von Lasern als Anregungslichtquellen eröffnete sowohl für die geräteseitig zu realisierende optische Detektion mit der wegen der hohen Intensität der Laser deutlich gesteigerten Empfindlichkeit der Messinstrumente als auch Farbstoff-seitig völlig neue Möglichkeiten der spektralen Kombination weit über den Bereich des Sichtbaren hinaus. Neben konventionellen Lasern nahmen und nehmen nahezu monochromatisch Licht emittierende Dioden (LEDs) und Festkörper-Laserdioden [Imasaka T., Diode lasers in analytical chemistry, Talanta, 48 (1999) 305-320] einen immer höheren Stellenwert bei der fluoreszenzbasierten Detektion in bioanalytischen Anwendungen ein.

Das Streben nach immer höherem Informationsgewinn bei bioanalytischen Verfahren findet seinen Ausdruck in Mehrfarb-Analysen, wie sie beispielsweise in der Durchfluß-Zytometrie [Lee L.G., NEAR-IR Dyes in Three Color Volumetrie Capillary Cytometry: Cell Analysis With 633- and 785-nm Laser Excitation, Cytometry, 21 (1995) 120-128], der DNA-Sequenzierung und verschiedenen PCR-Methoden (Roche's LightCycler) mittlerweile zum Laboralltag gehören. Anfänglich wurde hier auf Farbstoff-Kombinationen zurückgegriffen, die mittels Energietransfer von einem Donor auf verschiedene Akzeptoren bei Anregung durch ein und dieselbe (monochromatische) Lichtquelle spektral unterscheidbare Signale liefern. Beispiele hierfür sind die DNA-Sequenzer von Amersham (jetzt GE Healthcare) und ABI (jetzt Life Technologies), die Ende der 1990er Jahre auf den Markt kamen.

In der vorliegenden Beschreibung werden die Begriffe DNA gleich DNS (Desoxyribonukleinsäure) und RNA gleich RNS (Ribonukleinsäure) verwendet.

Ein alternativer Ansatz für Multiplex-Anwendungen stellt der Einsatz von Farbstoffen dar, die ohne Energietransfer zwischen Donor und Akzeptor eine spektrale Differenzierung zulassen. Beispiele hierfür finden sich in den seit ca. 2002 bekannten MegaStokes-Farbstoffen (EP 1 318 177 B1, EP 1 535 969 B1). Diese sind vorzugsweise auf eine Anregungswellenlänge zwischen 470 nm (blaugrüne LED) und 500 nm (488 nm-Ar-Ionen-Laser) zugeschnitten, da sie somit dem damaligen Stand der Technik bei Anregungslichtquellen am besten entsprachen.

Auch die Kombination von mehreren Anregungslichtquellen mit mehr als einem Chromphor je Lichtquelle hat mittlerweile Eingang in den Stand der Technik gefunden (Solexa, WO2007/135'368A2). Eine typische Anwendung ist hier das Next Generation Sequencing (NGS).

Mit der Verfügbarkeit von lichtintensiven, kurzwelligen Anregungsquellen wie UV-LEDs bzw. violetten Laserdioden verbreiterte sich auch die Basis geeigneter Fluorophore für bioanalytische Anwendungen. Ein Beispiel hierfür ist das Pacific Orange von Molecular Probes (US 8'158'801), das in Analogie zu den MegaStokes™-Farbstoffen in Kombination mit Cumarin-basierten Chromophoren Mehrfarbanalysen erlaubte, allerdings bei einer Anregung um 400 nm.

Der Erfindung liegt die Aufgabe zu Grunde, Fluoreszenzmarker auf der Basis von Benzoxazolen mit großem Stokes-Shift, mit hoher Photo- und Lagerstabilität sowie möglichst hoher Fluoreszenzquantenaus-beute zu schaffen.

Die vorliegende Erfindung beschreibt Marker-Farbstoffe auf der Basis von Benzoxazolen der allgemeinen Struktur mit n = 0 oder 1 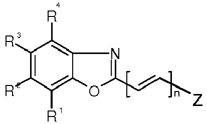 und ihre Salze, wobei Z entweder
Z = 4-Pyridyl- 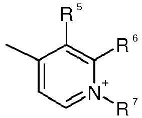 ist und die Struktur I gebildet ist oder
Z = 3-Pyridyl- 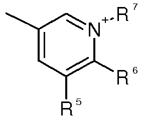 ist und die Struktur II gebildet ist oder
Z = 2-Pyridyl- 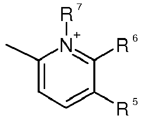 ist und die Struktur III gebildet ist
und R2 und / oder R7 je eine über einen Linker L gebundene reaktive Gruppe A zur kovalenten Bindung von Verbindungen der Strukturen I, II oder III mit einem zu markierenden Molekül K ist,
wobei A eine Amin- (-NH₂), Hydroxy- (-OH) oder Phosphoramidit-Funktion (-O-P-[O-CH₂-CH₂-CN]-N[(CH(CH₃)₂]₂), eine Carbonsäure (-COOH), ein davon abgeleiteter Alkyl- oder Aktiv-Ester (NHS-Ester, Sulfo-NHS-Ester, Tetrafluoro-Phenylester, p-Sulfo-Tetrafluoro-Phenylester), ein Carbonsäure-Hydrazid (-CONHNH₂) oder ein Carbonsäure-Amid (-CONHR12) mit R12 gleich -(CH₂)ₜ-Y, wobei
Y gleich -OH, -NH₂, -NH₃⁺, Maleimid (-N[CO-CH]₂), -NCS, -NCO, -NH-CO-CH₂-I, -NH-CO-CH₂-Br, -Azid (-N3), -Alkin (-CCH) oder-Phosphoramidit (-O-P-[O-CH₂-CH₂-CN]-N-[CH-(CH₃)₂]₂) und
K eine kovalent gebundene Komponente, ausgewählt aus der Gruppe Haptene (Moleküle, die ein unvollständiges Antigen darstellen und erst bei Bindung an Proteine bzw. Zellstrukturen die Wirkung eines Antigens zeigen), Proteine, Antikörper (Proteine, die als Reaktion auf Antigene gebildet werden), niedermolekulare Arzneistoffe (wirksame Bestandteile in Arzneimitteln, die aufgrund ihrer relativ geringen Molmasse bis etwa 800 g/ mol, im Gegensatz zu zum Beispiel Proteinen als sehr große Moleküle, in der Lage sind in Zellen einzudringen), Peptide (kleine bzw. kurzkettige Proteine bis etwa 100 verknüpften Aminosäuren), Nucleotide (Grundbausteine von Nucleinsäuren wie DNA oder RNA, die aus einem Phosphatteil, einem Monosaccharidteil und einem Nucleobasenteil wie Adenin, Guanin, Cytosin, Thymin oder Uracil bestehen), Nucleoside (Grundbausteine von Nucleinsäuren wie DNA oder RNA, die keinen Phosphatteil besitzen, sondern nur aus einem Monosaccharidteil und einem Nucleobasenteil bestehen), DNA-Oligomere (im Gegensatz zu DNA als Makromolekül Moleküle der Desoxyribonucleinsäure mit einer relativ geringen, nicht genau definierten Anzahl an Nucleotiden), Polymere (synthethische oder natürliche kettenförmige oder verzweigte chemische Verbindung aus sich wiederholenden Einheiten, den Monomeren. Polymere können auch als Copolymere aus mindestens zwei unterschiedlichen Monomeren in verschiedenen Mengenverhältnissen und Anordnungen bestehen) ist und
L gleich ein Linker ausgewählt aus einer Gruppe mit -(CH₂)ₛ-, -[(CH₂)ₘ-O]p-(CH₂)ₘ-, NR10-(CH₂)ₛ-, -O-(CH₂)ₛ-, -NR10-C(O)-(CH₂)ₛ-, -NR₁₀-C(O)-O-(CH₂)ₛ-oder -NR10-C(O)-NR11-(CH₂)ₛ- ist wobei R10 und R11 unabhängig voneinander Wasserstoff, Alkyl und Alkoxy-Alkyl (-[(CH₂)ₘ-O]ₚ- CH₃), ω-Sulfonsäure-Alkyl (-(CH₂)ᵣ-SO₃), m die Zahlen 2 - 5, p, r und s unabhängig voneinander die Zahlen von 1-10 darstellen, wobei R1, R3 - R6 unabhängig voneinander gleich Wasserstoff, Chlor, Brom oder ein Sulfonsäure-Rest sind,
im Falle dass R2 LA ist, ist L gleich NR10-(CH₂)ₛ-, -O-(CH₂)ₛ-, NR10-C(O)-(CH₂)₅-, -NR10-C(O)-O-(CH₂)ₛ-oder -NR10-C(O)-NR11-(CH₂)ₛ-,
im Falle dass R2 nicht LA ist, ist R2 gleich ω-Sulfonsäure-Alkoxy (-O-(CH₂)ᵣ- SO₃), Alkoxy, Poly-Alkoxy (-O-[(CH₂)ₘ-O]ₚ-CH₃), eine Amin-Funktion (-NR8R9) wobei R8 und R9 unabhängig voneinander Wasserstoff, -Alkyl, ω-Sulfonsäure-Alkyl (-(CH₂)ᵣ-SO₃), -CO-Alkyl, -CO-NR10-Alkyl, -CO-O-Alkyl,
im Falle dass R7 LA ist, ist L gleich -(CH₂)ₛ- oder -[(CH₂)ₘ-O]p-(CH₂)ₘ-,
im Falle dass R7 nicht LA ist, ist R7 gleich ω-Sulfonsäure-Alkyl (-(CH₂)ᵣ-SO₃),
im Falle dass in den Strukuren I und III R3 - R4 gleich -(CR13=CR14)₂- verbrückt sind, wobei R13 und R14 unabhängig voneinander Wasserstoff und /oder Sulfonsäure-Rest ist, sind R1, R2, R5, R6 gleich Wasserstoff oder ein Sulfonsäure-Rest, wobei mindestens einer von R1, R2, R13, R14 ein Sulfonsäure-Rest ist und R7 gleich -LA mit L gleich -(CH₂)ₛ- oder -[(CH₂)ₘ-O]p-(CH₂)ₘ-,
Im Falle dass in den Strukuren I und III R5 - R6 -(CR13=CR14)₂- verbrückt sind, wobei R13 und R14 unabhängig voneinander Wasserstoff und / oder Sulfonsäure-Rest ist, sind R1, R3, R4 gleich Wasserstoff, Chlor, Brom oder ein Sulfonsäure-Rest und R2 gleich Sulfonsäure-Alkoxy (-O-(CH₂)ᵣ-SO₃), -Alkoxy oder Poly-Alkoxy (-O-[(CH₂)ₘ-O]ₚ-CH₃ und R7 gleich -LA mit L gleich -(CH₂)ₛ- oder - [(CH₂)ₘ-O]ₚ-(CH₂)ₘ-,
im Falle dass in den Strukturen I und III R3 - R4 und R5 - R6 gleich -(CR13=CR14)₂- verbrückt sind, wobei R13 und R14 unabhängig voneinander Wasserstoff und / oder Sulfonsäure-Rest ist, sind R1, R2 gleich Wasserstoff oder ein Sulfonsäure-Rest ist, wobei mindestens einer von R1, R2, R13, R14 ein Sulfonsäure-Rest ist und R7 gleich-LA mit L gleich -(CH₂)ₛ-oder-[(CH₂)ₘ-O]ₚ-(CH₂)ₘ-. Verbindungen der Strukturen I bis III mit A in Form eines Aktiv-Esters werden beschrieben, wobei der Aktiv-Ester gleich ein NHS-Ester (N-Hydroxysuccinimidylester), ein Sulfo-NHS-Ester (Sulfo-Hydroxysuccinimidylester), ein TFP-Ester (Tetrafluoro-Phenylester) oder ein STP-Ester (p-Sulfo-Tetrafluoro-Phenylester) ist.

Auch werden Verbindungen der Strukturen I bis III beschrieben, dadurch gekennzeichnet, dass A in Form eines Carbonsäurederivats bzw. Verbindungen der Strukturen I bis III, dadurch gekennzeichnet, dass A in Form eines Phosphoramidits vorliegt, wobei A eine Gruppe der Formel 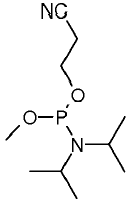 ist.

Die beschriebenen Verbindungen der Strukturen I bis III sind als Markerfarbstoffe auf der Basis von Benzoxazolen geeignet, wobei die zu markierenden Moleküle K über den Linker L und die reaktive Gruppe A an eine der Verbindungen der Strukturen I, II, III gebunden werden.

Weiterhin werden Verbindungen der Strukturen I und III beschrieben, dadurch gekennzeichnet, dass R3 und R4 und/oder R5 und R6 unabhängig miteinander verbunden sind und anellierte aromatische Ringe bilden, die mit Wasserstoff und / oder Sulfonsäure-Rest substituiert sind, wobei mindestens einer der Substituenten in der anellierten Benzoxazol-Teilstruktur ein Sulfonsäure-Rest ist.

Entsprechende Grundstrukturen, die meist nur in organischen Lösungsmittel löslich sind und keine Funktionalität aufweisen, die das kovalente Anbinden von beispielsweise Biomolekülen (chemische Verbindungen, die in Lebewesen vorkommen und essentiell sind unter anderem für den Stoffwechsel und Regulationsmechanismen) ermöglicht, sind in der Literatur beschrieben worden (z. B. R. F. Kubin et al. 1990, Laser Chemistry 10 (4): 247 - 58). Die Fluorophore müssen eine vom pH-Wert und anderen Umgebungseinflüssen (z. B. Polarität des Lösungsmittels) unabhängige Fluoreszenz aufweisen, in protischen Lösungsmitteln löslich sein sowie unter physiologischen Bedingungen mit einem geeigneten Biomolekül eine kovalente Bindung eingehen können.

Voraussetzung für den Einsatz als Fluoreszenzmarker ist das Eingehen einer kovalenten Bindung mit einem geeigneten Biomolekül, die über das Vorhandensein einer aktivierten Carbonsäure, einer aktivierten Hydroxy-Gruppe bzw. einer primären Aminofunktion realisiert wird. Für eine gute Wasserlöslichkeit ist die Anwesenheit polarer Substituenten, insbesondere von Sulfonsäure-Substituenten im Molekül erforderlich. Darüber hinaus beeinflussen die Sulfonsäure-Substituenten das Aggregationsverhalten (nicht-kovalente Dimerenbildung sowie nicht-kovalente Anbindung an Biomoleküle und Oberflächen). Sulfonsäure-Substituenten, welche direkt am Farbstoffgrundkörper gebunden sind, wirken auch auf die physiko-chemischen Eigenschaften der Farbstoffe in der Weise, dass die Absorptions- und Emissionswellenlängen hypsochrom verschoben werden und in der Regel eine signifikante Erhöhung der Quantenausbeute erzielt wird.

Die Benzoxazole der Strukturen I - III können als Farbstoffe zur optischen Markierung von organischen oder anorganischen Erkennungseinheiten, z. B. von Aminosäuren, Peptiden, Proteinen, Antigenen, Haptenen, Enzymsubstraten, Enzym-Cofaktoren, Biotin, Carotinoiden, Hormonen, Neurohormonen, Neurotransmittern, Wachstumsfaktoren, Lectinen, Toxinen, Kohlenhydraten, Oligosacchariden, Polysacchariden, Dextranen, Nucleinsäuren, Oligonucleotiden, DNA, RNA, biologischen Zellen, Lipiden, rezeptorbindenden Pharmaka oder organischen bzw. anorganischen polymeren Trägermaterialien verwendet werden.

Die Markierung der Erkennungseinheiten kann dabei durch die Ausbildung von ionischen oder van der Waals-Wechselwirkungen zwischen den Markern der Strukturen I - III und den zu markierenden Materialien erfolgen.

Weiterhin besteht auch die Möglichkeit, die Erkennungseinheit oder das Trägermaterial kovalent mit dem Fluorophor zu verbinden. Diese Kopplungsreaktion kann in wäßriger oder überwiegend wäßriger Lösung und vorzugsweise bei Raumtemperatur durchgeführt werden. Dabei entsteht eine Fluoreszenz-Sonde (Konjugat) zur qualitativen oder quantitativen Bestimmung von unterschiedlichen Biomaterialien bzw. anderen organischen und anorganischen Materialien.

Sowohl die Verbindungen der Strukturen I - III und davon abgeleitete Systeme können in optischen, insbesondere fluoreszenzoptischen, qualitativen und quantitativen Bestimmungsverfahren zur Diagnostik von Zelleigenschaften, in Biosensoren (point of care-Messungen), zur Erforschung des Genoms (DNA-Sequenzierung) und in Miniaturisierungstechnologien eingesetzt werden. Typische Anwendungen erfolgen in der Zytometrie und Zellsortierung, der Fluoreszenz-Korrelations-Spektroskopie (FCS), im Ultra-High-Throughput-Screening (UHTS), bei der multicolor Fluoreszenz-insitu-Hybridisierung (FISH) und in Mikroarrays (DNA- und Protein-Chips).

Dabei ist ein Mikroarray eine rasterartige Anordnung von auf mindestens einer Oberfläche immobilisierten Molekülen, die zum Studium von Rezeptor-Liganden-Wechselwirkungen verwendet werden können. Eine rasterartige Anordnung bedeutet mehr als zwei voneinander verschiedene Moleküle, welche sich innerhalb einer Fläche befinden und dort in unterschiedlichen, vorher definierten Regionen mit bekannter Position immobilisiert sind.

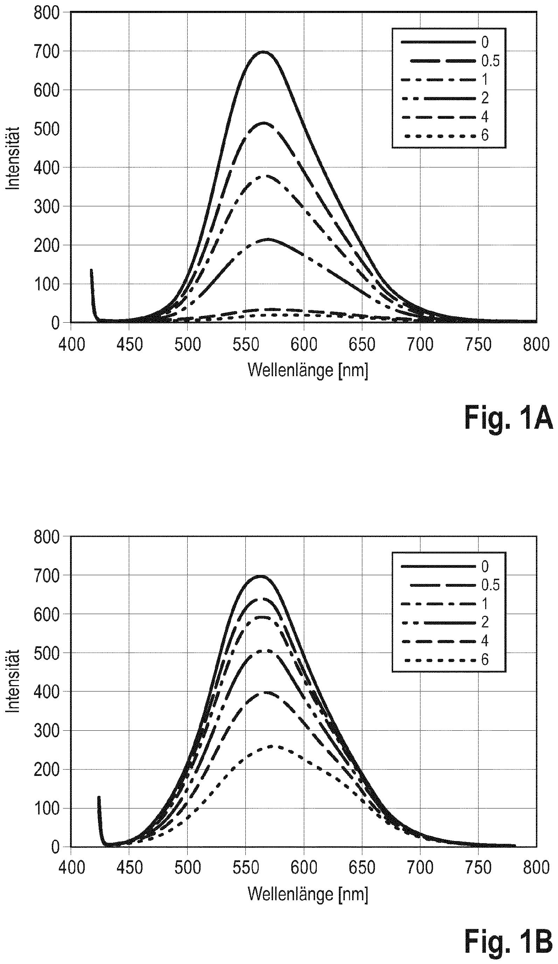

Ein Rezeptor ist ein Molekül, das eine Affinität zu einem gegebenen Liganden besitzt. Rezeptoren können natürlich vorkommende oder künstlich hergestellte Moleküle sein. Rezeptoren können in reiner Form oder gebunden an andere Spezies eingesetzt werden. Rezeptoren können kovalent oder nichtkovalent entweder direkt oder durch bestimmte Kopplungsvermittler an einen Bindungspartner angeknüpft werden.
Beispiele für Rezeptoren, die durch diese Erfindung detektiert werden können, schließen Agonisten und Antagonisten für Zell-Membran-Rezeptoren, Toxine und andere Giftstoffe, virale Epitope, Hormone wie Opiate und Steroide, Hormonrezeptoren, Peptide, Enzyme, Enzymsubstrate, als Kofaktoren agierende Wirkstoffe, Lektine, Zucker, Oligonukleotide, Nukleinsäuren, Oligosaccharide, Zellen, Zellfragmente, Gewebefragmente, Proteine und Antikörper ein, sind aber nicht auf die angeführten Stoffe beschränkt.
Ein Ligand ist ein Molekül, das von einem bestimmten Rezeptor erkannt wird. Beispiele für Liganden, die durch diese Erfindung detektiert werden können, schließen Agonisten und Antagonisten für Zell-Membran-Rezeptoren, Toxine und andere Giftstoffe, virale Epitope, Hormone wie Opiate und Steroide, Hormonrezeptoren, Peptide, Enzyme, Enzymsubstrate, als Kofaktoren agierende Wirkstoffe, Lektine, Zucker, Oligonukleotide, Nukleinsäuren, Oligosaccharide, Proteine und Antikörper ein, sind aber nicht auf die angeführten Stoffe beschränkt.
Durch die Darstellung von Benzoxazolen werden insbesondere nachfolgende Vorteile erreicht:
Bei geeigneter Substitution liegen die Absorptionsmaxima im Bereich der Emission der Laserwellenlänge von UV-Lasern (< 400 nm) und die Emissionsmaxima werden im Bereich zwischen 450 und 660 nm beobachtet. Abbildung 1 demonstriert die unterschiedlichen Emissionsbanden ausgewählter, erfindungsgemäßer Farbstoffe, wobei die Extinktion bei einer Anregungswellenlänge von 370 nm mindestens 75 % der maximalen Anregungseffizienz beträgt.

Ein weiterer Vorteil besteht darin, daß der hydrophile Charakter der unterschiedlich emittierenden Fluorophore nahezu beliebig gestaltet werden kann.

Erfindungsgemäße Verbindungen können wenigstens eine reaktive Gruppe A in Form eines Aktiv-Esters aufweisen, wobei der Aktiv-Ester gleich ein NHS-Ester (N-Hydroxysuccinimidylester), ein Sulfo-NHS-Ester (Sulfo-Hydroxysuccinimidylester), ein TFP-Ester (Tetrafluoro-Phenylester) oder ein STP-Ester (p-Sulfo-Tetrafluoro-Phenylester) ist, wie diese in der nachfolgenden Tabelle angegeben sind.

| A | Aktiv-Ester |
|---|---|
| 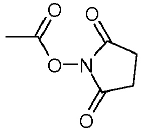 | NHS |
| 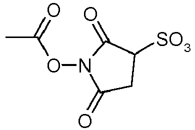 | Sulfo-NHS |
| 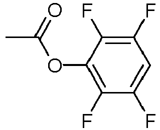 | TFP |
| 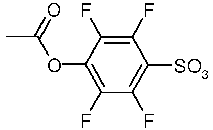 | STP |

Die erfindungsgemäßen Verbindungen können in weiteren Ausführungen A in Form eines Carbonsäurederivats aufweisen, wobei das Carbonsäurederivat ein Hydrazid, ein Amin, ein lod-Acetamid, ein Maleimid, ein Alkin oder ein Azid ist, wie diese in der nachstehenden Tabelle angegeben sind.

| A | Carbonsäureamid-Derivate |
|---|---|
| 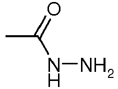 | Hydrazid |
| 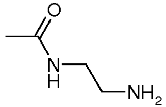 | Amin |
| 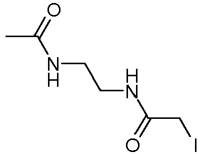 | Iod-acetamid |
| 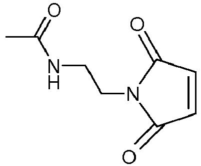 | Maleimid |
| 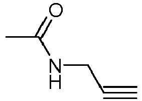 | Alkin |
| 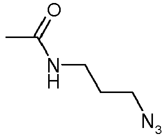 | Azid |

Die Erfindung ist nachfolgend anhand von Ausführungsbeispielen und Abbildungen näher ausgeführt. Es zeigen:
- Fig. 1A: Fluoreszenzlöschung durch Energietransfer für Verbindung 19/DYQ-1 A) Bindungspaar Streptavidin/Biotin,
- Fig. 1B: Fluoreszenzlöschung durch Energietransfer für Verbindung 19/DYQ-1 B) Blindprobe (Streptavidin/freie Säure),
- Fig. 2: Absorptions- und Emissionsspektren von Verbindung 22,
- Fig. 3: Absorptions- und Emissionsspektren von Verbindung 1,
- Fig. 4: Absorptions- und Emissionsspektren von Verbindung 17,
- Fig. 5: Absorptions- und Emissionsspektren von Verbindung 19,
- Fig. 6: Absorptions- und Emissionsspektren von Verbindung 21 und
- Fig. 7: Emissionsspektren ausgewählter erfindungsgemäßer Farbstoffe.

Die Kurvenverläufe in den Figuren 1A bis 7 sind den tatsächlichen Messwerten möglichst genau angepasst.

### Ausführungsbeispiel 1:

### 1-(5-Carboxy-pentyl)-4-[6-(3-sulfonato-propoxy)-benzooxazol-2-yl]-pyridinium Betain

### Vorstufe 1: 2-Pyridin-4-yl-benzooxazol-6-ol

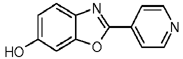

2,0 g 4-Aminoresorcinol-Hydrochlorid, 1,52 g Isonicotinsäure und 5 g Polyphosphorsäure-trimethylsilylester werden in 20 ml 1,2-Dichlorbenzen und 2,11 ml Ethyl-diisopropylamin für 6 Stunden auf 180°C erhitzt. Nach dem Abkühlen wird mit 70 ml Dichlormethan suspendiert und durch Zugabe von 50 ml 1 N NaOH-Lösung neutralisiert. Im Ultraschallbad wird solange behandelt, bis ein hellgrauer feiner Niederschlag vorliegt, welcher filtriert, mit wenig Wasser gewaschen und getrocknet wird. Ausbeute: 745 mg (28%) (C₁₂H₈N₂O₂; 212,21 g/mol)
MS ESI - (m/z): 211 (base, [M-H]⁻)

### Vorstufe 2: 1-(5-Ethoxycarbonyl-pentyl)-4-(6-hydroxy-benzooxazol-2-yl)-pyridinium Bromid

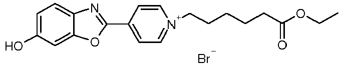

445 mg Vorstufe 1 werden mit 500 mg 6-Bromhexansäureethylester in 8 ml DMF 24 Stunden bei 150°C gerührt. Nach dem Abkühlen wird mit 40 ml Diethylether ein Öl gefällt, welches verworfen wird. Das organische Lösungsmittel wird abdestilliert und der Rückstand durch RP-Chromatografie gereinigt.
Ausbeute: 605 mg (64%) (C₂₀H₂₃N₂O₄⁺Br⁻; 435,32 g/mol)
MS ESI + (m/z): 355 (base, [M]⁺)

### Vorstufe 3: 1-(5-Carboxy-pentyl)-4-(6-hydroxy-benzooxazol-2-yl)-pyridinium Bromid

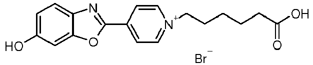

600 mg Vorstufe 2 werden in 3 M HCl 1 Stunde am Rückfluß gekocht. Nach Abkühlen wird die Lösung mit Natriumhydrogencarbonat neutralisiert. Die Reinigung erfolgt durch RP-Chromatografie.
Ausbeute: 480 mg (85%) (C₁₈H₁₉N₂O₄⁺ Br⁻; 407,26 g/mol)
MS ESI + (m/z): 327 (base, [M]⁺)
UV-Vis in PBS: λₘₐₓ: 385 nm; λₑₘ: 495 nm; ε= 19.400 l/mol*cm; QY: 0,02

### Verbindung 1:

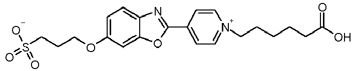

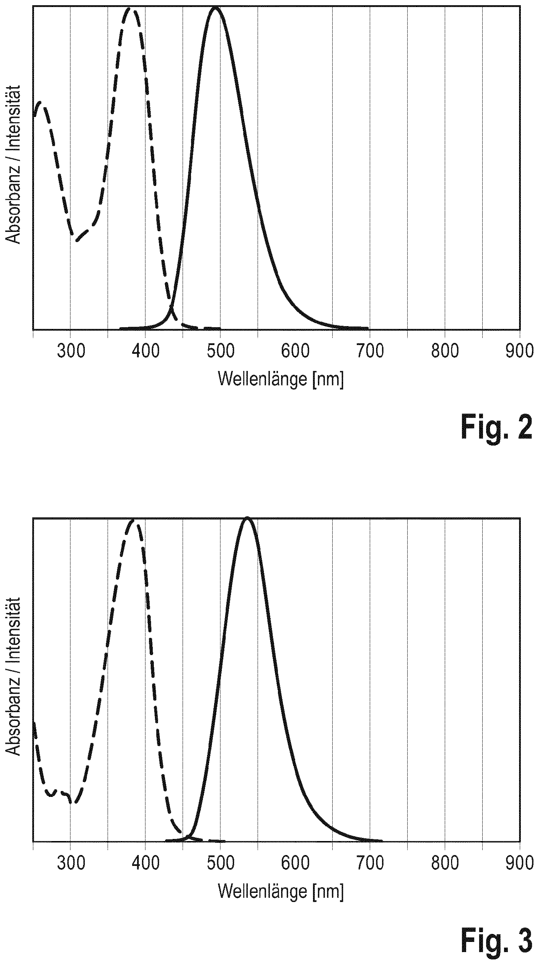

300 mg Vorstufe 3 werden in 3 ml DMF mit 118 mg Propansulton und 70 mg Kaluimhydrogencarbonat bei 100°C 5 Stunden gerührt. Zur Esterspaltung wird die gelartige Mischung mit 20 ml 3 M HCl versetzt und 1 Stunde am Rückfluß gekocht. Nach Neutralisation mit Natriumhydrogencarbonat wird über RP-Chromatografie gereinigt.
Ausbeute: 125 mg (42%) (C₂₁H₂₄N₂O₇S; 448,50 g/mol)
MS ESI- (m/z): 447 (base, [M-H]⁻); 895 (30%, [2M-H]⁻)
MS ESI + (m/z): 449 (base, [M + H]⁺); 471(15%, [M + Na]⁺)
UV-Vis in PBS: λₘₐₓ: 383 nm; λₑₘ: 516 nm (vgl. Fig. 3); ε = 22.000 l/mol*cm; QY: 0,70
¹H-NMR 400 MHz (d6-DMSO) δ (ppm): 12,05 (S, 1H, COOH); 9,23 (D, 2H, Pyridyl-H); 8,69 (D, 2H, Pyridyl-H); 7,86 (D, 1H, Benzoxazol-H); 7,51 (D, 1H, Benzoxazol-H); 7,14 (D, 1H, Benzoxazol-H); 4,66 (T, 2H, N-CH₂-); 4,23 (T, 2H, O-CH₂-); 2,59 (M, 2H, -CH₂-COOH); 2,24 (M, 2H, -CH₂-SO3⁻); 2,06 (M, 2H, - CH₂-); 1,97 (M, 2H, -CH₂-); 1,56 (M, 2H, -CH₂-); 1,33 (M, 2H, -CH₂-)

### Ausführungsbeispiel 2:

### 1-(5-Carboxy-pentyl)-4-[5-chloro-6-(3-sulfonato-propoxy)-benzooxazol-2-yl]-pyridinium Betain

### Vorstufe 1: 5-Chloro-2-pyridin-4-yl-benzooxazol-6-ol

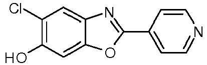

2,42 g 4-Amino-6-chloro-1,3-benzendiol-Hydrochlorid, 1,52 g Isonicotinsäure und 5 g Polyphosphorsäure-trimethylsilylester werden in 20 ml 1,2-Dichlorbenzen und 2,11 ml Ethyl-diisopropylamin für 6 Stunden auf 180°C erhitzt. Nach dem Abkühlen wird mit 70 ml Dichlormethan suspendiert und durch Zugabe von 50 ml 1 N NaOH-Lösung neutralisiert. Im Ultraschallbad wird solange behandelt, bis ein hellgrauer feiner Niederschlag vorliegt, welcher filtriert, mit wenig Wasser gewaschen und getrocknet wird. Ausbeute: 560 mg (18%) (C₁₂H₇ClN₂O₂; 246,65 g/mol)
MS ESI - (m/z): 245 (base, [M-H]⁻)
Vorstufe 2: 4-(5-Chloro-6-hydroxy-benzooxazol-2-yl)-1-(5-ethoxycarbonyl-pentyl)-pyridinium Bromid

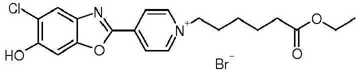

520 mg Vorstufe 1 werden mit 500 mg 6-Bromhexansäureethylester in 8 ml DMF 24 Stunden bei 150°C gerührt. Nach dem Abkühlen wird mit 40 ml Diethylether ein Öl gefällt, welches verworfen wird. Das organische Lösungsmittel wird abdestilliert und der Rückstand durch RP-Chromatografie gereinigt.
Ausbeute: 720 mg (72%) (C₂₀H₂₂ClN₂O₄⁺Br⁻; 469,76 g/mol)
MS ESI + (m/z): 389 (base, [M]⁺)

### Vorstufe 3: 1-(5-Carboxy-pentyl)-4-(5-chloro-6-hydroxy-benzooxazol-2-yl)-pyridinium Bromid

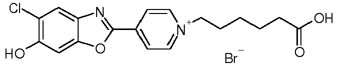

650 mg Vorstufe 2 werden in 3 M HCl 1 Stunde am Rückfluß gekocht. Nach Abkühlen wird die Lösung mit Natriumhydrogencarbonat neutralisiert. Die Reinigung erfolgt durch RP-Chromatografie.
Ausbeute: 490 mg (80%) (C₁₈H₁₈ClN₂O₄⁺ Br⁻; 441,71 g/mol)
MS ESI + (m/z): 361 (base, [M]⁺)
UV-Vis in PBS: λₘₐₓ: 465 nm; λₑₘ: 655 nm; ε= 19.800 l/mol*cm; QY: 0,01

### Verbindung 2:

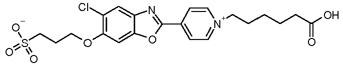

325 mg Vorstufe 3 werden in 3 ml DMF mit 118 mg Propansulton und 70 mg Kaluimhydrogencarbonat bei 100°C 5 Stunden gerührt. Zur Esterspaltung wird die gelartige Mischung mit 20 ml 3 M HCl versetzt und 1 Stunde am Rückfluß gekocht. Nach Neutralisation mit Natriumhydrogencarbonat wird über RP-Chromatografie gereinigt.
Ausbeute: 135 mg (38%) (C₂₁H₂₃ClN₂O₇S; 482,94 g/mol)
MS ESI + (m/z): 483 (base, [M+H]⁺); 485 (30%, [M+H ³⁷Cl]⁺)
UV-Vis in PBS: λₘₐₓ: 375 nm; λₑₘ: 505 nm; ε = 22.000 l/mol*cm; QY: 0,92

### Ausführungsbeispiel 3:

### 1-(5-Carboxy-pentyl)-4-[5-chloro-6-(3-sulfonato-propoxy)-benzooxazol-2-yl]-pyridinium Betain

### Vorstufe 1: 5,7-Dichloro-2-(3-chloro-pyridin-4-yl)-benzooxazol-6-ol

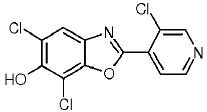

2,85 g 4-Amino-2,6-chloro-benzen-1,3-diol-Hydrochlorid, 1,95 g 3-Chloropyridin-4-carbonsäure und 5 g Polyphosphor-säure-trimethylsilylester werden in 20 ml 1,2-Dichlorbenzen und 2,11 ml Ethyl-diisopropylamin für 6 Stunden auf 180°C erhitzt. Nach dem Abkühlen wird mit 70 ml Dichlormethan suspendiert und durch Zugabe von 50 ml 1 N NaOH-Lösung neutralisiert. Im Ultraschallbad wird solange behandelt, bis ein hellgrauer feiner Niederschlag vorliegt, welcher filtriert, mit wenig Wasser gewaschen und getrocknet wird. Ausbeute: 930 mg (23%) (C₁₂H₅Cl₃N₂O₂; 315,54 g/mol)
MS ESI - (m/z): 314 (base, [M-H]⁻); 316 (90%, [M-H]⁻); 318 (30%, [M-H]⁻)

### Vorstufe 2: 3-Chloro-4-(5,7-dichloro-6-hydroxy-benzooxazol-2-yl)-1-(5-ethoxycarbonyl-pentyl)-pyridinium Bromid

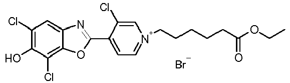

670 mg Vorstufe 1 werden mit 500 mg 6-Bromhexansäureethylester in 8 ml DMF 24 Stunden bei 150°C gerührt. Nach dem Abkühlen wird mit 40 ml Diethylether ein Öl gefällt, welches verworfen wird. Das organische Lösungsmittel wird abdestilliert und der Rückstand durch RP-Chromatografie gereinigt.
Ausbeute: 570 mg (50%) (C₂₀H₂₀Cl₃N₂O₄⁺ Br⁻; 538,65 g/mol)
MS ESI - (m/z): 456 (base, [M-H]⁻); 458 (95%, [M-H]⁻); 460 (40%, [M-H]⁻)

### Vorstufe 3: 1-(5-Carboxy-pentyl)-3-chloro-4-(5,7-dichloro-6-hydroxy-benzooxazol-2-yl)-pyridinium Bromid

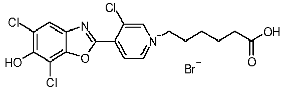

560 mg Vorstufe 2 werden in 3 M HCl 1 Stunde am Rückfluß gekocht. Nach Abkühlen wird die Lösung mit Natriumhydrogencarbonat neutralisiert. Die Reinigung erfolgt durch RP-Chromatografie.
Ausbeute: 430 mg (82%) (C₁₈H₁₆Cl₃N₂O₄⁺ Br⁻; 510,60 g/mol)
MS ESI - (m/z): 428 (base, [M-H]⁻); 430 (95%, [M-H]⁻); 432 (35%, [M-H]⁻)
UV-Vis in PBS: λₘₐₓ: 462 nm; λₑₘ: 650 nm; ε= 19.200l/mol*cm; QY: 0,04

### Verbindung 3:

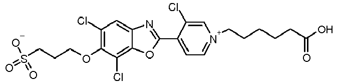

380 mg Vorstufe 3 werden in 3 ml DMF mit 118 mg Propansulton und 70 mg Kaluimhydrogencarbonat bei 100°C 5 Stunden gerührt. Zur Esterspaltung wird die gelartige Mischung mit 20 ml 3 M HCl versetzt und 1 Stunde am Rückfluß gekocht. Nach Neutralisation mit Natriumhydrogencarbonat wird über RP-Chromatografie gereinigt.
Ausbeute: 110 mg (28%) (C₂₁H₂₁Cl₃N₂O₇S; 551,83 g/mol)
MS ESI + (m/z): 551 (base, [M+H]⁻); 553 (80%, [M+H]⁻); 555 (40%, [M+H]⁻)
UV-Vis in PBS: λₘₐₓ: 345 nm; λₑₘ: 506 nm; ε = 20.000 l/mol*cm; QY: 0,14

### Ausführungsbeispiel 4:

### 1-(5-Carboxy-pentyl)-3-chloro-4-[6-(3-sulfonato-propoxy)-benzooxazol-2-yl)-pyridinium Betain

### Vorstufe 1: 2-(3-Chloro-pyridin-4-yl)-benzooxazol-6-ol

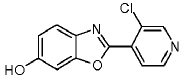

2,0 g 4-Aminoresorcinol-Hydrochlorid, 1,95 g 3-Chlorpyridin-4-carbonsäure und 5 g Polyphosphorsäure-trimethylsilylester werden in 20 ml 1,2-Dichlorbenzen und 2,11 ml Ethyl-diisopropylamin für 6 Stunden auf 180°C erhitzt. Nach dem Abkühlen wird mit 70 ml Dichlormethan suspendiert und durch Zugabe von 50 ml 1 N NaOH-Lösung neutralisiert. Im Ultraschallbad wird solange behandelt, bis ein hellgrauer feiner Niederschlag vorliegt, welcher filtriert, mit wenig Wasser gewaschen und getrocknet wird. Ausbeute: 910 mg (30%) (C₁₂H₇ClN₂O₂; 246,65 g/mol)
MS ESI - (m/z): 245 (base, [M-H]⁻)

### Vorstufe 2: 3-Chloro-1-(5-ethoxycarbonyl-pentyl)-4-(6-hydroxy-benzooxazol-2-yl)-pyridinium Bromid

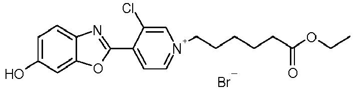

520 mg Vorstufe 1 werden mit 500 mg 6-Bromhexansäureethylester in 8 ml DMF 24 Stunden bei 150°C gerührt. Nach Abkühlen wird mit 40 ml Diethylether ein Öl gefällt, welches verworfen wird. Das organische Lösungsmittel wird abdestilliert und der Rückstand durch RP-Chromatografie gereinigt.
Ausbeute: 590 mg (60%) (C₂₀H₂₂ClN₂O₄⁺Br⁻; 469,76 g/mol)
MS ESI - (m/z): 387 (base, [M-H]⁻)

### Vorstufe 3: 1-(5-Carboxy-pentyl)-3-chloro-4-(6-hydroxy-benzooxazol-2-yl)-pyridinium Bromid

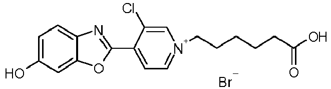

570 mg Vorstufe 2 werden in 3 M HCl 1 Stunde am Rückfluß gekocht. Nach Abkühlen wird die Lösung mit Natriumhydrogencarbonat neutralisiert. Die Reinigung erfolgt durch RP-Chromatografie.
Ausbeute: 430 mg (80%) (C₁₈H₁₈ClN₂O₄⁺Br⁻; 441,71 g/mol)
MS ESI + (m/z): 361 (base, [M]⁺)
UV-Vis in PBS: λₘₐₓ: 465 nm; λₑₘ: 655 nm; ε= 18.900l/mol*cm; QY: 0,02

### Verbindung 4:

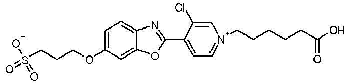

325 mg Vorstufe 3 werden in 3 ml DMF mit 118 mg Propansulton und 70 mg Kaluimhydrogencarbonat bei 100°C 5 Stunden gerührt. Zur Esterspaltung wird die gelartige Mischung mit 20 ml 3 M HCl versetzt und 1 Stunde am Rückfluß gekocht. Nach Neutralisation mit Natriumhydrogencarbonat wird über RP-Chromatografie gereinigt.
Ausbeute: 140 mg (40%) (C₂₁H₂₃ClN₂O₇S; 482,94 g/mol)
MS ESI + (m/z): 483 (base, [M+H]⁺); 485 (30%, [M+H ³⁷Cl]⁺)
UV-Vis in PBS: λₘₐₓ: 375 nm; λₑₘ: 510 nm; ε = 21.800 l/mol*cm; QY: 0,85

### Ausführungsbeispiel 5:

### 1-(5-Carboxy-pentyl)-3chloro-4-[5-sulfonato-6-(3-sulfonato-propoxy)-benzooxazol-2-yl]-pyridinium Natriumsalz

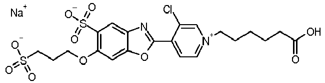

120 mg Verbindung 4 werden in 2 ml Oleum 20%ig gelöst und 24 Stunden bei 60°C gerührt. Die Mischung wird auf Eis gegossen und die entstehende Lösung mit Natriumhydrogencarbonat neutralisiert. Die Reinigung erfolgt durch RP-Chromatografie.
Ausbeute: 60 mg (41%) (C₂₁H₂₂ClN₂O₁₀S₂Na; 584,99 g/mol)
MS ESI - (m/z): 561 (base, [M]⁻)
UV-Vis in PBS: λₘₐₓ: 365 nm; λₑₘ: 495 nm; ε = 21.000 l/mol*cm; QY: 0,85

### Ausführungsbeispiel 6:

### 1-[2-(2-{2-[2-(2-Carboxy-ethoxy)-ethoxy]-ethoxy}-ethoxy)-ethyl]-2-[6-(2-{2-[2-(2-methoxy-ethoxy)-ethoxy]-ethoxy}-ethoxy)-benzooxazol-2-yl]-pyridinium Chlorid

### Vorstufe 1: 2-Pyridin-2-yl-benzooxazol-6-ol

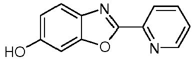

2,0 g 4-Aminoresorcinol-Hydrochlorid, 1,52 g Pyridin-2-carbonsäure und 5 g Polyphosphorsäure-trimethylsilylester werden in 20 ml 1,2-Dichlorbenzen und 2,11 ml Ethyl-diisopropylamin für 6 Stunden auf 180°C erhitzt. Nach dem Abkühlen wird mit 70 ml Dichlormethan suspendiert und durch Zugabe von 50 ml 1 N NaOH-Lösung neutralisiert. Im Ultraschallbad wird solange behandelt, bis ein hellgrauer feiner Niederschlag vorliegt, welcher filtriert, mit wenig Wasser gewaschen und getrocknet wird. Ausbeute: 745 mg (28%) (C₁₂H₈N₂O₂; 212,21 g/mol)
MS ESI - (m/z): 211 (base, [M-H]⁻)

### Vorstufe 2: 1-[2-(2-{2-[2-(2-Carboxy-ethoxy)-ethoxy]-ethoxy}-ethoxy)-ethyl]-2-(6-hydroxy-benzooxazol-2-yl)-pyridinium Toluensulfonat

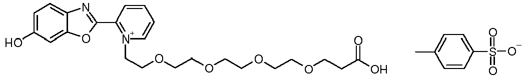

445 mg Vorstufe 1 werden mit 1,1 g 3-[2-(2-{2-[2-(Toluen-4-sulfonyloxy)-ethoxy]-ethoxy}-ethoxy)-ethoxy]-propionsäure-tert-butylester in 12 ml DMF 24 Stunden bei 150°C gerührt. Das Lösungsmittel wird abdestilliert, der Rückstand mehrfach mit Heptan digeriert und das überstehende Lösungsmittel verworfen. Der ölige Rückstand durch RP-Chromatografie gereinigt.
Ausbeute: 570 mg (43%) (C₂₃H₂₉N₂O₈⁺C₇H₇O₃S⁻; 632,70 g/mol)
MS ESI + (m/z): 461 (base, [M]⁺)
UV-Vis in PBS: λₘₐₓ: 372 nm; λₑₘ: 515 nm; ε=18.500l/mol*cm; QY: 0,02

### Verbindung 6:

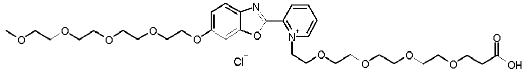

450 mg Vorstufe 2 werden in 5 ml DMF mit 300 mg 1-Bromo-2-{2-[2-(2-methoxy-ethoxy)-ethoxy]-ethoxy}-ethan und 70 mg Kaluimhydrogencarbonat bei 100°C 16 Stunden gerührt. Zur Esterspaltung wird die Mischung mit 20 ml 3 M HCl versetzt und 1 Stunde am Rückfluß gekocht. Nach Abstumpfen mit Natriumhydrogencarbonat wird über RP-Chromatografie gereinigt.
Ausbeute: 180 mg (37%) (C₃₂H₄₇N₂O₁₂⁺Cl⁻; 687,19 g/mol)
MS ESI + (m/z): 651 (base, [M]⁺
UV-Vis in PBS: λₘₐₓ: 375 nm; λₑₘ: 500 nm; ε = 20.200l/mol*cm; QY: 0,72

### Ausführungsbeispiel 7:

### 1-(5-Carboxy-pentyl)-4-[6-(5-carboxy-pentyloxy)-benzooxazol-2-yl]-pyridinium Betain

### Vorstufe: 2-Pyridin-4-yl-benzooxazol-6-ol

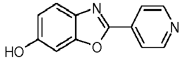

2,0 g 4-Aminoresorcinol-Hydrochlorid, 1,52 g Isonicotinsäure und 5 g Polyphosphorsäure-trimethylsilylester werden in 20 ml 1,2-Dichlorbenzen und 2,11 ml Ethyl-diisopropylamin für 6 Stunden auf 180°C erhitzt. Nach dem Abkühlen wird mit 70 ml Dichlormethan suspendiert und durch Zugabe von 50 ml 1 N NaOH-Lösung neutralisiert. Im Ultraschallbad wird solange behandelt, bis ein hellgrauer feiner Niederschlag vorliegt, welcher filtriert, mit wenig Wasser gewaschen und getrocknet wird. Ausbeute: 745 mg (28%) (C₁₂H₈N₂O₂; 212,21 g/mol)
MS ESI - (m/z): 211 (base, [M-H]⁻)

### Verbindung 7:

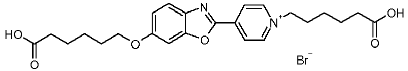

500 mg Vorstufe werden in 8 ml DMF mit 1,6 g 6-Bromhexansäureethylester und 235 mg Kaluimhydrogencarbonat bei 100°C 5 Stunden und bei 150°C 16 Stunden gerührt. Das organische Lösungsmittel wird abdestilliert, die ölige Mischung mit 30 ml 3 M HCl versetzt und 1 Stunde am Rückfluß gekocht. Nach Neutralisation mit Natriumhydrogencarbonat wird über RP-Chromatografie gereinigt.
Ausbeute: 710 mg (58%) (C₂₄H₂₉N₂O₆⁺Br⁻; 521,41 g/mol)
MS ESI + (m/z): 441 (base, [M]⁺)
MS ESI- (m/z): 439 (base, [M-2 H]⁻)
UV-Vis in PBS: λₘₐₓ: 383 nm; λₑₘ: 516 nm; ε= 22.000 l/mol*cm; QY: 0,70

### Ausführungsbeispiel 8:

### 1-(5-Carboxy-pentyl)-4-[6-(5-carboxy-pentyloxy)-5-sulfonato-benzooxazol-2-yl]-pyridinium Betain

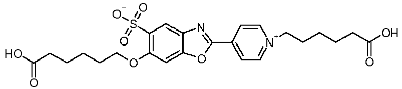

500 mg Verbindung 7 werden in 3 ml Oleum 20%ig gelöst und 24 Stunden bei 60°C gerührt. Die Mischung wird auf Eis gegossen und die entstehende Lösung mit Natriumhydrogencarbonat neutralisiert. Die Reinigung erfolgt durch RP-Chromatografie.
Ausbeute: 260 mg (52%) (C₂₄H₂₈N₂O₉S; 520,56 g/mol)
MS ESI - (m/z): 519 (base, [M-H]⁻)
UV-Vis in PBS: λₘₐₓ: 370 nm; λₑₘ: 497 nm; ε = 20.700 l/mol*cm; QY: 0,69

### Ausführungsbeispiel 9:

### 1-(5-Carboxy-pentyl)-4-[6-(3-sulfonato-propoxy)-benzooxazol-2-yl]-chinolinium Betain

### Vorstufe 1: 2-Chinolin-4-yl-benzooxazol-6-ol

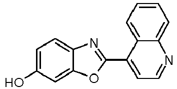

0,933 g 4-Aminoresorcinol-Hydrochlorid, 1,0 g 4-Chinolincarbonsäure und 4 g Polyphosphorsäure-trimethylsilylester werden in 10 ml 1,2-Dichlorbenzen und 0,99 ml Ethyl-diisopropylamin für 6 Stunden auf 180°C erhitzt. Nach dem Abkühlen wird mit 70 ml Dichlormethan suspendiert und durch Zugabe von 40 ml 1 N NaOH-Lösung neutralisiert. Im Ultraschallbad wird solange behandelt, bis ein hellgrauer feiner Niederschlag vorliegt, welcher filtriert, mit wenig Wasser und wenig Dichlormethan gewaschen und getrocknet wird.
Ausbeute: 595 mg (39%) (C₁₆H₁₀N₂O₂; 262,27 g/mol)
MS ESI - (m/z): 261 (base, [M-H]⁻)

### Vorstufe 2: 3-(2-Chinolin-4-yl-benzooxazol-6-yloxy)-propan-1-sulfonsäure Natriumsalz

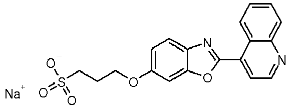

590 mg Vorstufe 1 werden in 6 ml trockenem DMF mit 110 mg Natriumhydrid (60%ig in Mineralöl) portionsweise versetzt und 30 Minuten bei RT gerührt. Nach Zugabe von 412 mg Propansulton wird 1 Stunde bei 120°C gerührt. Das Lösungsmittel wird abdestilliert und der Rückstand mit Diethylether behandelt, wobei ein Niederschlag ausfällt. Dieser wird filtriert, mit Diethylether gewaschen und getrocknet.
Ausbeute: 730 mg (80%) (C₁₉H₁₅N₂O₅SNa; 406,41 g/mol)
MS ESI - (m/z): 383 (base, [M]⁻)

### Verbindung 9:

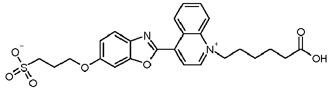

700 mg Vorstufe 2 werden mit 450 mg 6-Bromhexansäureethylester in 15 ml DMF 48 Stunden bei 150°C gerührt. Nach Abkühlen wird mit Diethylether ein öliger Niederschlag gefällt. Zur Esterspaltung wird dieser in 20 ml 3 M HCl 1 Stunde am Rückfluß gekocht und nach Abkühlen wird die Lösung mit Natriumhydrogencarbonat neutralisiert. Die Reinigung erfolgt durch RP-Chromatografie.
Ausbeute: 435 mg (52%) (C₂₅H₂₆N₂O₇S; 498,56 g/mol)
MS ESI - (m/z): 497 (base, [M-H]⁻)
UV-Vis in PBS: λₘₐₓ: 415 nm; λₑₘ: 568 nm; ε = 18.900 l/mol*cm; QY: 0,04

### Ausführungsbeispiel 10:

### 1-(5-Carboxy-pentyl)-4-[5-sulfonato-6-(3-sulfonato-propoxy)-benzooxazol-2-yl]-chinolinium Natriumsalz

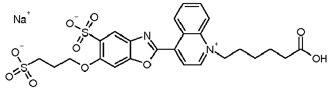

130 mg Verbindung 9 werden in 2 ml Oleum 20%ig gelöst und 24 Stunden bei 60°C gerührt. Die Mischung wird auf Eis gegossen und die entstehende Lösung mit Natriumhydrogencarbonat neutralisiert. Die Reinigung erfolgt durch RP-Chromatografie.
Ausbeute: 70 mg (47%) (C₂₅H₂₅N₂O₁₀S₂Na; 600,60 g/mol)
MS ESI - (m/z): 577 (base, [M]⁻)
UV-Vis in PBS: λₘₐₓ: 403 nm; λₑₘ: 549 nm; ε = 18.000 l/mol*cm; QY: 0,15

### Ausführungsbeispiel 11:

### 1-[2-(2-{2-[2-(2-Carboxy-ethoxy)-ethoxy]-ethoxy}-ethoxy)-ethyl]-2-[6-(3-sulfonato-propoxy)-benzooxazol-2-yl]-chinolinium Betain

### Vorstufe 1: 2-Chinolin-2-yl-benzooxazol-6-ol

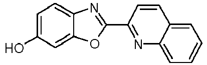

0,933 g 4-Aminoresorcinol-Hydrochlorid, 1,0 g Chinolin-2-carbonsäure und 4 g Polyphosphorsäure-trimethylsilylester werden in 10 ml 1,2-Dichlorbenzen und 0,99 ml Ethyl-diisopropylamin für 6 Stunden auf 180°C erhitzt. Nach dem Abkühlen wird mit 70 ml Dichlormethan suspendiert und durch Zugabe von 40 ml 1 N NaOH-Lösung neutralisiert. Im Ultraschallbad wird solange behandelt, bis ein hellgrauer feiner Niederschlag vorliegt, welcher filtriert, mit wenig Wasser und wenig Dichlormethan gewaschen und getrocknet wird.
Ausbeute: 640 mg (42%) (C₁₆H₁₀N₂O₂; 262,27 g/mol)
MS ESI - (m/z): 261 (base, [M-H]⁻)

### Vorstufe 2: 3-(2-Chinolin-2-yl-benzooxazol-6-yloxy)-propan-1-sulfonsäure Natriumsalz

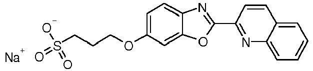

590 mg Vorstufe 1 werden in 6 ml trockenem DMF mit 110 mg Natriumhydrid (60%ig in Mineralöl) portionsweise versetzt und 30 Minuten bei RT gerührt. Nach Zugabe von 412 mg Propansulton wird 1 Stunde bei 120°C gerührt. Das Lösungsmittel wird abdestilliert und der Rückstand mit Diethylether behandelt, wobei ein Niederschlag ausfällt. Dieser wird filtriert, mit Diethylether gewaschen und getrocknet.
Ausbeute: 690 mg (75%) (C₁₉H₁₅N₂O₅SNa; 406,41 g/mol)
MS ESI - (m/z): 383 (base, [M]⁻)

### Verbindung 11:

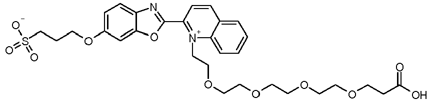

600 mg Vorstufe 2 werden mit 990 mg 3-[2-(2-{2-[2-(Toluen-4-sulfonyloxy)-ethoxy]-ethoxy}-ethoxy)-ethoxy]-propionsäure-tert-butylester in 15 ml DMF 48 Stunden bei 150°C gerührt. Das Lösungsmittel wird abdestilliert, der Rückstand mehrfach mit Heptan digeriert und das überstehende Lösungsmittel verworfen. Der ölige Rückstand durch RP-Chromatografie gereinigt.
Ausbeute: 360 mg (38%) (C₃₀H₃₆N₂O₁₁S; 632,69 g/mol)
MS ESI - (m/z): 631 (base, [M-H]⁻)
UV-Vis in PBS: λₘₐₓ: 405 nm; λₑₘ: 552 nm; ε = 18.200 l/mol*cm; QY: 0,06

### Ausführungsbeispiel 12:

### 1-(5-Carboxy-pentyl)-4-(5-sulfonato-naphtho[1,2-d]oxazol-2-yl)-chinolinium Betain

### Vorstufe: 2-Chinolin-4-yl-naphtho[1,2-d]oxazol-5-sulfonsäure Natriumsalz

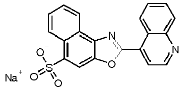

700 mg 1-Amino-2-naphthol-4-sulfonsäure Natriumsalz, 500 mg Chinolin-4-carbonsäure und 2 g Polyphosphorsäure-trimethylsilylester werden in 7 ml 1,2-Dichlorbenzen für 12 Stunden auf 180°C erhitzt. Nach dem Abkühlen wird mit 20 ml Dichlormethan suspendiert und durch Zugabe von 20 ml 1 N NaOH-Lösung neutralisiert. Im Ultraschallbad wird solange behandelt, bis sich der klebrige Niederschlag weitgehend gelöst hat. Die wäßrige Phase wird abgetrennt und über RP-Chromatografie gereinigt.
Ausbeute: 115 mg (10%) (C₂₀H₁₁N₂O₄SNa; 398,38 g/mol)
MS ESI - (m/z): 375 (base, [M]⁻)

### Verbindung 12:

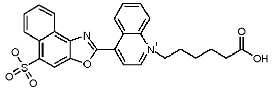

80 mg Vorstufe werden mit 90 mg 6-Bromhexansäureethylester in 5 ml DMF 48 Stunden bei 150°C gerührt. Das Lösungsmittel wird abdestilliert und der Rückstand in 10 ml 3 M HCl 1 Stunde am Rückfluß gekocht. Nach Abkühlen wird die Lösung mit Natriumhydrogencarbonat neutralisiert. Die Reinigung erfolgt durch RP-Chromatografie.
Ausbeute: 12 mg (12%) (C₂₆H₂₂N₂O₆S; 490,54 g/mol)
MS ESI - (m/z): 489 (base, [M-H]⁻)
UV-Vis in PBS: λₘₐₓ: 414 nm; λₑₘ: 555 nm; ε = 19.000 l/mol*cm; QY: 0,43

### Ausführungsbeispiel 13:

### 1-(5-Carboxy-pentyl)-3-{2-[6-(3-sulfonato-propoxy)-benzooxazol-2-yl]-vinyl}-pyridinium Betain

### Vorstufe 1: 2-(2-Pyridin-3-yl-vinyl)-benzooxazol-6-ol

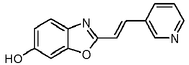

2,0 g 4-Aminoresorcinol-Hydrochlorid, 1,85 g trans-3-(3-Pyridyl)-acrylsäure und 5 g Polyphosphorsäure-trimethylsilylester werden in 20 ml 1,2-Dichlorbenzen und 2,11 ml Ethyl-diisopropylamin für 6 Stunden auf 180°C erhitzt. Nach dem Abkühlen wird mit 50 ml 1 N NaOH-Lösung neutralisiert. Im Ultraschallbad wird solange behandelt, bis ein hellgrauer feiner Niederschlag vorliegt, welcher filtriert, mit wenig Wasser sowie wenig Dichlormethan gewaschen und getrocknet wird. Ausbeute: 830 mg (28%) (C₁₄H₁₀N₂O₂; 238,25 g/mol)
MS ESI - (m/z): 237 (base, [M-H]⁻)
UV-Vis in PBS: λₘₐₓ: 340 nm; λₑₘ: 455 nm; ε = 15.500 l/mol*cm; QY: 0,40

### Vorstufe 2: 3-[2-(2-Pyridin-3-yl-vinyl)-benzooxazol-6-yloxy]-propan-1-sulfonsäure Natriumsalz

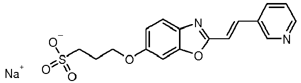

800 mg Vorstufe 1 werden in 30 ml trockenem DMF mit 150 mg Natriumhydrid (60%ig in Mineralöl) portionsweise versetzt und 1 Stunde bei RT gerührt. Nach Zugabe von 420 mg Propansulton wird 1 Stunde bei 120°C gerührt. Nach Abkühlen wird die Fällung durch Zugabe von Diethylether vervollständigt. Der Niederschlag wird in Methanol aufgenommen, filtriert, das Lösungsmittel abdestilliert und der Rückstand getrocknet.
Ausbeute: 1,1 g (80%) (C₁₇H₁₅N₂O₅SNa; 382,37 g/mol)
MS ESI - (m/z): 359 (base, [M]⁻)
UV-Vis in PBS: λₘₐₓ: 339 nm; λₑₘ: 445 nm; ε = 17.000l/mol*cm; QY: 0,74

### Verbindung 13:

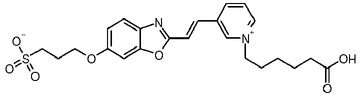

600 mg Vorstufe 2 werden mit 400 mg 6-Bromhexansäureethylester in 20 ml DMF 8 Stunden bei 150°C gerührt. Das Lösungsmittel wird abdestilliert und der Rückstand in 25 ml 3 M HCl 1 Stunde am Rückfluß gekocht. Nach Abkühlen wird die Lösung mit Natriumhydrogencarbonat neutralisiert. Die Reinigung erfolgt durch RP-Chromatografie.
Ausbeute: 75 mg (10%) (C₂₃H₂₆N₂O₇S; 474,54 g/mol)
MS ESI - (m/z): 473 (base, [M-H]⁻)
UV-Vis in PBS: λₘₐₓ: 360 nm; λₑₘ: 530 nm; ε = 17.000l/mol*cm; QY: 0,02

### Ausführungsbeispiel 14:

### 1-(5-Carboxy-pentyl)-4-(6-methoxy-benzooxazol-2-yl)-pyridinium Bromid

### Vorstufe: 6-Methoxy-2-pyridin-4-yl-benzooxazol

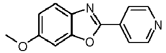

1,4 g 2-Amino-5-methoxy-phenol, 1,25 g Isonicotinsäure und 10 g Polyphosphorsäure-trimethylsilylester werden in 18 ml 1,2-Dichlorbenzen für 4 Stunden auf 180°C erhitzt. Nach dem Abkühlen wird mit 70 ml Dichlormethan suspendiert und durch Zugabe von 100 ml 1 N NaOH-Lösung neutralisiert. Im Ultraschallbad wird solange behandelt, bis ein hellgrauer feiner Niederschlag vorliegt, welcher filtriert, mit wenig Wasser gewaschen und getrocknet wird. Ausbeute: 725 mg (32%) (C₁₃H₁₀N₂O₂; 226,24 g/mol)
MS ESI + (m/z): 227 (base, [M + H]⁺)

### Verbindung 14:

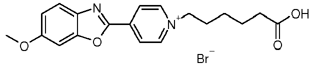

500 mg Vorstufe werden mit 1 g 6-Bromhexansäureethylester in 4 ml 1,2-Dichlorbenzen und 4 ml DMF 24 Stunden bei 150°C gerührt. Nach Abkühlen wird mit 100 ml Diethylether ein Öl gefällt, welches in 10 ml 3 M HCl 1 Stunde am Rückfluß gekocht wird. Nach Abkühlen wird die Lösung mit Natriumhydrogencarbonat neutralisiert. Die Reinigung erfolgt durch RP-Chromatografie.
Ausbeute: 130 mg (14%) (C₁₉H₂₁N₂O₄⁺ Br⁻; 421,29 g/mol)
MS ESI + (m/z): 420 (base, [M]⁺)
UV-Vis in PBS: λₘₐₓ: 380 nm; λₑₘ: 511 nm; ε = 19.800 l/mol*cm; QY: 0,50

### Ausführungsbeispiel 15:

### 1-(5-Carboxy-pentyl)-4-(6-methoxy-5-sulfonato-benzooxazol-2-yl)-pyridinium Betain

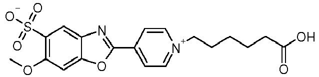

100 mg Verbindung 14 werden in 2 ml Oleum 20%ig gelöst und 2 Stunden bei RT gerührt. Die Mischung wird auf Eis gegossen und die entstehende Lösung mit Natriumhydrogencarbonat neutralisiert. Die Reinigung erfolgt durch RP-Chromatografie.
Ausbeute: 55 mg (54%) (C₁₉H₂₀N₂O₇S; 420,44 g/mol)
MS ESI - (m/ z): 419 (base, [M-H]⁻)
UV-Vis in PBS: λₘₐₓ: 369 nm; λₑₘ: 495 nm; ε = 19.200 l/mol*cm; QY: 0,70

### Ausführungsbeispiel 16:

### 1-(3-Hydroxy-propyl)-4-(6-methoxy-benzooxazol-2-yl)-pyridinium Bromid

### Vorstufe: 6-Methoxy-2-pyridin-4-yl-benzooxazol

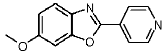

1,4 g 2-Amino-5-methoxy-phenol, 1,25 g Isonicotinsäure und 10 g Polyphosphorsäure-trimethylsilylester werden in 18 ml 1,2-Dichlorbenzen für 4 Stunden auf 180°C erhitzt. Nach dem Abkühlen wird mit 70 ml Dichlormethan suspendiert und durch Zugabe von 100 ml 1 N NaOH-Lösung neutralisiert. Im Ultraschallbad wird solange behandelt, bis ein hellgrauer feiner Niederschlag vorliegt, welcher filtriert, mit wenig Wasser gewaschen und getrocknet wird. Ausbeute: 725 mg (32%) (C₁₃H₁₀N₂O₂; 226,24 g/mol)
MS ESI + (m/z): 227 (base, [M + H]⁺)

### Verbindung 16:

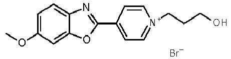

500 mg 2,21mmol Vorstufe werden mit 800 mg Essigsäure-3-bromopropylester in 4 ml 1,2-Dichlorbenzen und 4 ml DMF 24 Stunden bei 150°C gerührt. Nach Abkühlen wird mit 100 ml Diethylether ein Öl gefällt, welches in 10 ml 3 M HCl 1 Stunde am Rückfluß gekocht wird. Nach Abkühlen wird die Lösung mit Natriumhydrogencarbonat neutralisiert. Die Reinigung erfolgt durch RP-Chromatografie.
Ausbeute: 130 mg (16%) (C₁₆H₁₇N₂O₃⁺ Br⁻; 365,22 g/mol)
MS ESI + (m/z): 285 (base, [M]⁺)
UV-Vis in PBS: λₘₐₓ: 379 nm; λₑₘ: 510 nm; ε = 20.000l/mol*cm; QY: 0,50

### Ausführungsbeispiel 17:

### 4-[6-(4-Carboxy-butyrylamino)-benzooxazol-2-yl]-1-(3-sulfonato-propyl)-pyridinium Betain

### Vorstufe: 4-(2-Pyridin-4-yl-benzooxazol-6-ylcarbamoyl)-buttersäure

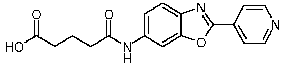

2,5 g 4-(4-Amino-3-hydroxy-phenylcarbamoyl)-buttersäure, 1,3 g Isonicotinsäure und 10 g Polyphosphorsäure-trimethylsilylester werden in 18 ml 1,2-Dichlorbenzen für 4 Stunden auf 180°C erhitzt. Nach dem Abkühlen wird mit 70 ml Dichlormethan suspendiert und durch Zugabe von 100 ml 1 N NaOH-Lösung neutralisiert. Im Ultraschallbad wird solange behandelt, bis ein hellgrauer feiner Niederschlag vorliegt, welcher filtriert, mit wenig Wasser gewaschen und getrocknet wird.
Ausbeute: 750 mg (22%) (C₁₇H₁₅N₃O₄; 325,33 g/mol)
MS ESI - (m/z): 324 (base, [M - H]⁻)

### Verbindung 17:

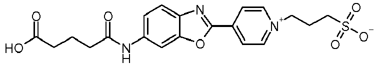

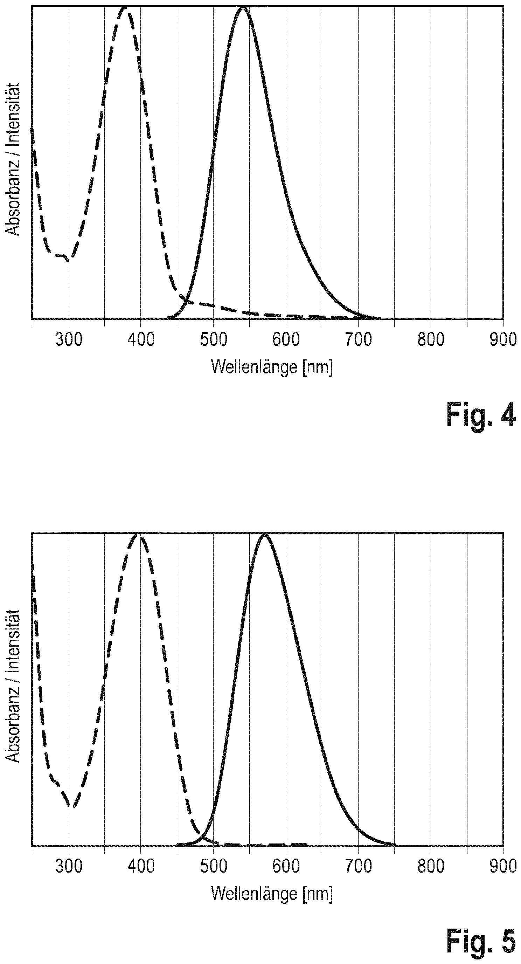

500 mg Vorstufe werden mit 375 mg Propansulton in 4 ml DMF 8 Stunden bei 120°C gerührt. Das Lösungsmittel wird abdestilliert und der Rückstand mittels RP-Chromatografie gereinigt.
Ausbeute: 330 mg (48%) (C₂₀H₂₁N₃O₇S; 447,47 g/mol)
MS ESI - (m/z): 446 (base, [M-H]⁻)
UV-Vis in PBS: λₘₐₓ: 375 nm; λₑₘ: 543 nm (vgl. Fig. 4); ε = 16.000 l/mol*cm; QY: 0,62

### Ausführungsbeispiel 18:

### 1-(5-Carboxy-pentyl)-4-(6-ethoxycarbonylamino-benzooxazol-2-yl)-pyridinium Bromid

### Vorstufe: (2-Pyridin-4-yl-benzooxazol-6-yl)-carbamidsäure-ethylester

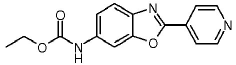

2,0 g (4-Amino-3-hydroxy-phenyl)-carbamidsäure-ethylester, 1,3 g Isonicotinsäure und 10 g Polyphosphorsäure-trimethylsilylester werden in 18 ml 1,2-Dichlorbenzen für 4 Stunden auf 180°C erhitzt. Nach dem Abkühlen wird mit 50 ml Dichlormethan suspendiert und durch Zugabe von 100 ml 1 N NaOH-Lösung neutralisiert. Im Ultraschallbad wird solange behandelt, bis ein hellgrauer feiner Niederschlag vorliegt, welcher filtriert, mit wenig Wasser gewaschen und getrocknet wird.
Ausbeute: 340 mg (12%) (C₁₅H₁₃N₃O₃; 283,29 g/mol)
MS ESI + (m/z): 284 (base, [M + H]⁺)

### Verbindung 18:

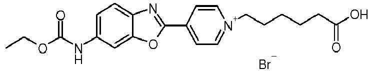

340 mg Vorstufe werden mit 530 mg 6-Bromhexansäureethylester in 3 ml 1,2-Dichlorbenzen und 3 ml DMF 24 Stunden bei 150°C gerührt. Nach Abkühlen wird mit 100 ml Diethylether ein Öl gefällt, welches in einem Gemisch aus 30 ml Aceton und 20 ml konzentrierter Natriumhydrogencarbonatlösung bei 50°C 4 Stunden gerührt wird. Die Mischung wird mit 5%iger HBr neutralisiert. Die Reinigung erfolgt durch RP-Chromatografie.
Ausbeute: 80 mg (14%) (C₂₁H₂₄N₃O₅⁺ Br⁻; 478,34 g/mol)
MS ESI + (m/z): 398 (base, [M]⁺)
UV-Vis in PBS: λₘₐₓ: 383 nm; λₑₘ: 543 nm; ε = 17.000 l/mol*cm; QY: 0,54

### Ausführungsbeispiel 19:

### 4-{6-[3-(5-Carboxy-pentyl)-ureido]-benzooxazol-2-yl}-1-(3-sulfonato-propyl)-pyridinium Betain

### Vorstufe: 6-[3-(2-Pyridin-4-yl-benzooxazol-6-yl)-ureido]-hexansäure

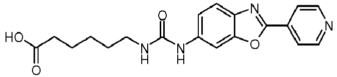

4,0 g 6-[3-(4-Amino-3-hydroxy-phenyl)-ureido]-hexansäure, 1,75 g Isonicotinsäure und 13 g Polyphosphorsäure-trimethylsilylester werden in 20 ml 1,2-Dichlorbenzen für 4 Stunden auf 180°C erhitzt. Nach dem Abkühlen wird mit 70 ml Dichlormethan suspendiert und durch Zugabe von 120 ml 1 N NaOH-Lösung neutralisiert. Im Ultraschallbad wird solange behandelt, bis ein hellgrauer feiner Niederschlag vorliegt, welcher filtriert, mit wenig Wasser gewaschen und getrocknet wird.
Ausbeute: 420 mg (8%) (C₁₉H₂₀N₄O₄; 368,40 g/mol)
MS ESI - (m/z): 367 (base, [M - H]⁻)

### Verbindung 19:

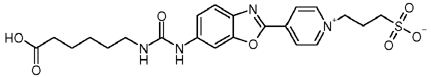

400 mg Vorstufe werden mit 265 mg Propansulton in 4 ml DMF 8 Stunden bei 120°C gerührt. Das Lösungsmittel wird abdestilliert und der Rückstand mittels RP-Chromatografie gereinigt.
Ausbeute: 195 mg (37%) (C₂₂H₂₆N₄O₇S; 490,54 g/mol)
MS ESI - (m/z): 489 (base, [M]⁺)
UV-Vis in PBS: λₘₐₓ: 397 nm; λₑₘ: 572 nm (vgl. Fig. 5); ε = 20.000 l/mol*cm; QY: 0,28

### Ausführungsbeispiel 20:

### 4-{6-[Bis-(3-sulfonato-propyl)-amino]-benzooxazol-2-yl}-1-(5-carboxy-pentyl)-pyridinium Natriumsalz

### Vorstufe: 3-[(2-Pyridin-4-yl-benzooxazol-6-yl)-(3-sulfonato-propyl)-amino]-propansulfonsäure di-Natriumsalz

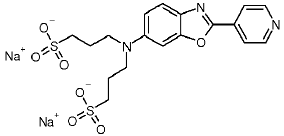

2,0 g 3-[(4-Amino-3-hydroxy-phenyl)-(3-sulfonato-propyl)-amino]-propansulfonsäure di-Natriumsalz, 600 mg Isonicotinsäure und 6 g Polyphosphorsäure-trimethylsilylester werden in 15 ml 1,2-Dichlorbenzen für 4 Stunden auf 180°C erhitzt. Nach dem Abkühlen wird mit 50 ml Dichlormethan verdünnt und durch Zugabe von 65 ml 1 N NaOH-Lösung neutralisiert. Im Ultraschallbad wird solange behandelt, bis sich der klebrige Niederschlag weitgehend gelöst hat. Die wäßrige Phase wird abgetrennt und über RP-Chromatografie gereinigt.
Ausbeute: 675 mg (28%) (C₁₈H₁₉N₃O₇S₂Na₂; 499,48 g/mol)
MS ESI - (m/z): 226,7 (base, [M]²⁻)

### Verbindung 20:

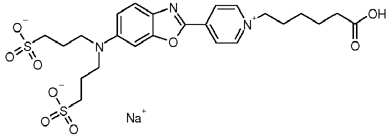

500 mg Vorstufe werden mit 450 mg 6-Bromhexansäureethylester in 3 ml 1,2-Dichlorbenzen und 5 ml DMF 24 Stunden bei 150°C gerührt. Nach Abkühlen wird mit 50 ml Diethylether ein Öl gefällt, welches in 10 ml 3 M HCl 1 Stunde am Rückfluß gekocht wird. Nach Abkühlen wird die Lösung mit Natriumhydrogencarbonat neutralisiert. Die Reinigung erfolgt durch RP-Chromatografie.
Ausbeute: 115 mg (19%) (C₂₄H₃₀N₃O₉S₂Na; 591,64 g/mol)
MS ESI - (m/z): 568 (base, [M]⁻)
UV-Vis in PBS: λₘₐₓ: 491 nm; λₑₘ: 666 nm; ε = 17.700 l/mol*cm; QY: 0,06

### Ausführungsbeispiel 21:

### 4-{6-[1-(3-sulfonato-propyl)-3-(5-Carboxy-pentyl)-ureido]-benzooxazol-2-yl}-1-(3-sulfonato-propyl)-pyridinium di-Natriumsalz

### Vorstufe: 4-{6-[1-(3-sulfonato-propyl)-3-(5-Carboxy-pentyl)-ureido]-benzooxazol-2-yl}-pyridin Natriumsalz

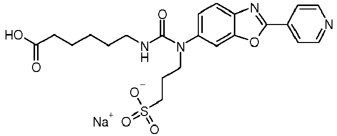

5,0 g 6-[3-(4-Amino-3-hydroxy-phenyl)-ureido]-hexansäure, 2,1 g Isonicotinsäure und 15 g Polyphosphor-säure-trimethylsilylester werden in 25 ml 1,2-Dichlorbenzen für 4 Stunden auf 180°C erhitzt. Nach dem Abkühlen wird mit 70 ml Dichlormethan suspendiert und durch Zugabe von 120 ml 1 N NaOH-Lösung neutralisiert. Im Ultraschallbad wird solange behandelt, bis sich der klebrige Niederschlag weitgehend gelöst hat. Die wäßrige Phase wird abgetrennt und über RP-Chromatografie gereinigt.
Ausbeute: 660 mg (11%) (C₂₂H₂₅N₄O₇SNa; 512,52 g/mol)
MS ESI - (m/z): 489 (base, [M]⁻)

### Verbindung 21:

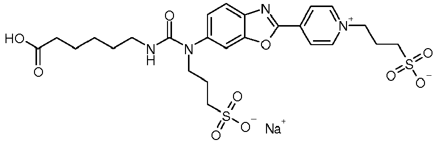

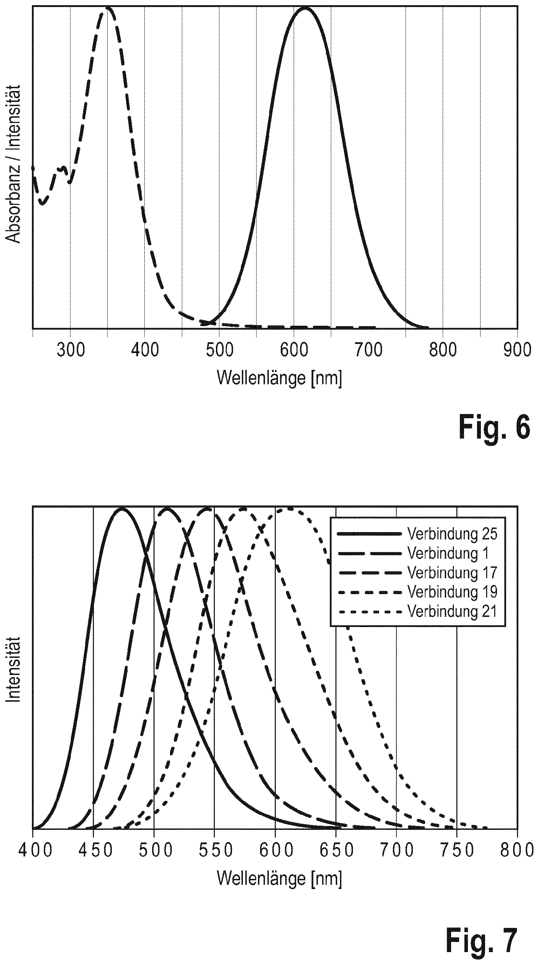

660 mg Vorstufe werden mit 300 mg Propansulton in 8 ml DMF 8 Stunden bei 120°C gerührt. Das Lösungsmittel wird abdestilliert und der Rückstand mittels RP-Chromatografie gereinigt.
Ausbeute: 280 mg (33%) (C₂₅H₃₁N₄O₁₀S₂Na; 634,66 g/mol)
MS ESI - (m/z): 611 (base, [M + Na]⁻)
UV-Vis in PBS: λₘₐₓ: 353 nm; λₑₘ: 614 nm (vgl Fig. 6); ε = 16.000 l/mol*cm; QY: 0,29

### Ausführungsbeispiel 22:

### 1-(5-Carboxy-pentyl)-4-(4,7-disulfonato-naphtho[1,2-d]oxazol-2-yl)-pyridinium Natriumsalz

### Vorstufe: 2-Pyridin-4-yl-naphtho[1,2-d]oxazol-4,7-disulfonsäure di-Natriumsalz

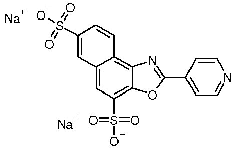

3,6 g 4-Amino-3-hydroxy-naphthalen-2,7-disulfonsäure di-Natriumsalz, 1,22 g Isonicotinsäure und 10 g Polyphosphorsäure-trimethylsilylester werden in 18 ml 1,2-Dichlorbenzen für 4 Stunden auf 180°C erhitzt. Nach dem Abkühlen wird mit 50 ml Dichlormethan verdünnt und durch Zugabe von 100 ml 1 N NaOH-Lösung neutralisiert. Im Ultraschallbad wird solange behandelt, bis sich der klebrige Niederschlag weitgehend gelöst hat. Die wäßrige Phase wird abgetrennt und über RP-Chromatografie gereinigt.
Ausbeute: 735 mg (16%) (C₁₆H₈N₂O₇S₂Na₂; 450,36 g/mol)
MS ESI - (m/z): 202 (base, [M]²⁻)

### Verbindung 22:

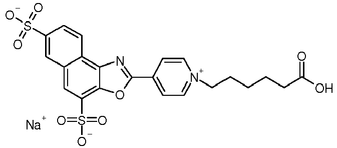

600 mg Vorstufe werden mit 600 mg 6-Bromhexansäureethylester in 3 ml 1,2-Dichlorbenzen und 5 ml DMF 24 Stunden bei 150°C gerührt. Nach Abkühlen wird mit 50 ml Diethylether ein Öl gefällt, welches in 10 ml 3 M HCl 1 Stunde am Rückfluß gekocht wird. Nach Abkühlen wird die Lösung mit Natriumhydrogencarbonat neutralisiert. Die Reinigung erfolgt durch RP-Chromatografie.
Ausbeute: 80 mg (11%) (C₂₂H₁₉N₂O₉S₂Na; 542,52 g/mol)
MS ESI - (m/z): 520 (base, [M]⁻)
UV-Vis in PBS: λₘₐₓ: 376 nm; λₑₘ: 484 nm (vgl. Fig. 2); ε = 15.700 l/mol*cm; QY: 0,75

### Ausführungsbeispiel 23:

### 1-(5-Carboxy-pentyl)-2-(4,7-disulfonato-naphtho[1,2-d]oxazol-2-yl)-Chinolinium Natriumsalz

### Vorstufe: 2-Chinolin-2-yl-naphtho[1,2-d]oxazol-4,7-disulfonsäure di-Natriumsalz

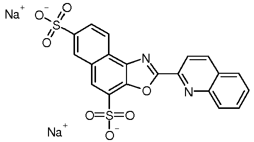

1 g 4-Amino-3-hydroxy-naphthalen-2,7-disulfonsäure di-Natriumsalz, 480 mg Chinolin-2-carbonsäure und 4,0 g Polyphosphorsäure-trimethylsilylester werden in 6 ml 1,2-Dichlorbenzen für 9 Stunden auf 180°C erhitzt. Nach dem Abkühlen wird mit 20 ml Dichlormethan suspendiert und durch Zugabe von 40 ml 1 N NaOH-Lösung neutralisiert. Die wäßrige Phase wird abgetrennt und über RP-Chromatografie gereinigt.
Ausbeute: 250 mg (18%) (C₂₀H₁₀N₂O₇S₂Na₂; 500,42 g/mol)
MS ESI - (m/z): 227 (base, [M]²⁻)

### Verbindung 23:

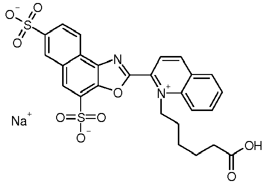

190 mg Vorstufe werden mit 170 mg 6-Bromhexansäureethylester in 2 ml DMF 48 Stunden bei 150°C gerührt. Das Lösungsmittel wird abdestilliert und der Rückstand in 5 ml 3 M HCl 1 Stunde am Rückfluß gekocht. Nach Abkühlen wird die Lösung mit Natriumhydrogencarbonat neutralisiert. Die Reinigung erfolgt durch RP-Chromatografie.
Ausbeute: 36 mg (16%) (C₂₆H₂₁N₂O₉S₂Na; 592,58 g/mol)
MS ESI - (m/z): 569 (base, [M]⁻)
UV-Vis in PBS: λₘₐₓ: 494 nm; λₑₘ: 515 nm; ε = 18.000 l/mol*cm; QY: 0,75

### Ausführungsbeispiel 24:

### 1-(5-Carboxy-pentyl)-4-(4,7-disulfonato-naphtho[1,2-d]oxazol-2-yl)-Chinolinium Natriumsalz

### Vorstufe: 2-Chinolin-4-yl-naphtho[1,2-d]oxazol-4,7-disulfonsäure di-Natriumsalz

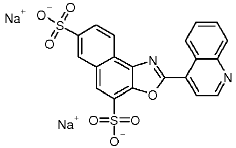

1 g 4-Amino-3-hydroxy-naphthalen-2,7-disulfonsäure di-Natriumsalz, 480 mg Chinolin-4-carbonsäure und 4,0 g Polyphosphorsäure-trimethylsilylester werden in 6 ml 1,2-Dichlorbenzen für 9 Stunden auf 180°C erhitzt. Nach dem Abkühlen wird mit 20 ml Dichlormethan suspendiert und durch Zugabe von 40 ml 1 N NaOH-Lösung neutralisiert. Die wäßrige Phase wird abgetrennt und über RP-Chromatografie gereinigt.
Ausbeute: 205 mg (15%) (C₂₀H₁₀N₂O₇S₂Na₂; 500,42 g/mol)
MS ESI - (m/z): 227 (base, [M]²⁻)

### Verbindung 24:

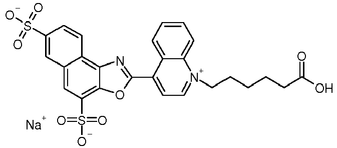

190 mg Vorstufe werden mit 170 mg 6-Bromhexansäureethylester in 2 ml DMF 48 Stunden bei 150°C gerührt. Das Lösungsmittel wird abdestilliert und der Rückstand in 5 ml 3 M HCl 1 Stunde am Rückfluß gekocht. Nach Abkühlen wird die Lösung mit Natriumhydrogencarbonat neutralisiert. Die Reinigung erfolgt durch RP-Chromatografie.
Ausbeute: 31 mg (14%) (C₂₆H₂₁N₂O₉S₂Na; 592,58 g/mol)
MS ESI - (m/z): 569 (base, [M]⁻)
UV-Vis in PBS: λₘₐₓ: 404 nm; λₑₘ: 535 nm; ε = 18.000 l/mol*cm; QY: 0,73

### Ausführungsbeispiel 25:

### 1-(5-Carboxy-pentyl)-2-(4,7-disulfonato-naphtho[1,2-d]oxazol-2-yl)-pyridinium Natriumsalz

### Vorstufe: 2-Pyridin-2-yl-naphtho[1,2-d]oxazol-4,7-disulfonsäure di-Natriumsalz

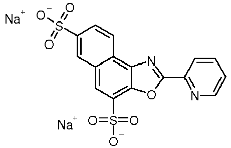

5 g 4-Amino-3-hydroxy-naphthalen-2,7-disulfonsäure di-Natriumsalz, 1,7 g 2-Picolinsäure und 14 g Polyphosphorsäure-trimethylsilylester werden in 22 ml 1,2-Dichlorbenzen für 4 Stunden auf 180°C erhitzt. Nach dem Abkühlen wird mit 70 ml Dichlormethan verdünnt und durch Zugabe von 140 ml 1 N NaOH-Lösung neutralisiert. Im Ultraschallbad wird solange behandelt, bis sich der klebrige Niederschlag weitgehend gelöst hat. Die wäßrige Phase wird abgetrennt und über RP-Chromatografie gereinigt.
Ausbeute: 740 mg (12%) (C₁₆H₈N₂O₇S₂Na₂; 450,36 g/mol)
MS ESI - (m/z): 202 (base, [M]²⁻)

### Verbindung 25:

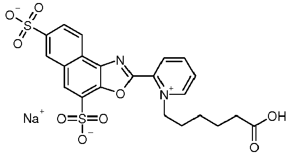

700 mg Vorstufe werden mit 700 mg 6-Bromhexansäureethylester in 4 ml 1,2-Dichlorbenzen und 6 ml DMF (Dimethylformamid) 24 Stunden bei 150°C gerührt. Nach Abkühlen wird mit 70 ml Diethylether ein Öl gefällt, welches in 15 ml 3 M HCl 1 Stunde am Rückfluß gekocht wird. Nach Abkühlen wird die Lösung mit Natriumhydrogencarbonat neutralisiert. Die Reinigung erfolgt durch RP-Chromatografie.
Ausbeute: 75 mg (9%) (C₂₂H₁₉N₂O₉S₂Na; 542,52 g/mol)
MS ESI - (m/z): 520 (base, [M]⁻)
UV-Vis in PBS: λₘₐₓ: 368 nm; λₑₘ: 474 nm; ε = 13.200 l/mol*cm; QY: 0,85

### Ausführungsbeispiele 26-49 (NHS-Ester)

Die NHS-Ester der gemäß der Ausführungsbeispiele 1 bis 15 und 17 bis 25 erhaltenen Verbindungen werden nach der folgenden Methode hergestellt:
0,25 mmol der Carbonsäure werden in 3 ml DMF gelöst. Bei 0°C werden 90 mg TSTU (N,N,N',N' - Tetramethyl-O-(N-succinimidyl) uronium tetrafluoroborat) und 52 µl DIPEA (Diisopropylethylamin) zugegeben und es wird bei RT 20 Minuten gerührt. Im Falle der Verbindung 7 werden 200 mg TSTU und 120 µl DIPEA eingesetzt. Das Lösungsmittel wird im Vakuum abdestilliert und der Rückstand über eine RP-Säule gereinigt.

In der nachfolgenden Tabelle sind zeilenweise die NHS-Ester aufgeführt (letzte Spalte), die aus einer jeweiligen Ausgangsverbindung (zweite Spalte) bei Anwendung der vorstehend beschriebenen Methode erhalten werden. Die Nummern 1 bis 15 und 17 bis 25 der Ausgangsverbindungen entsprechen den Nummern der Ausführungsbeispiele 1 bis 15 und 17 bis 25.

| Ausführungsbeispiel | Ausgangsverbindung | Summenformel | Molmasse [g/mol] | NHS-Ester |
|---|---|---|---|---|
| 26 | 1 | C₂₅H₂₇N₃O₉S | 545,57 | 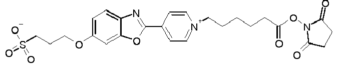 |
| 27 | 2 | C₂₅H₂₆ClN₃O₉S | 580,02 | 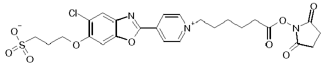 |
| 28 | 3 | C₂₅H₂₄Cl₃N₃O₉S | 648,91 | 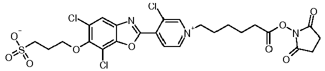 |
| 29 | 4 | C₂₅H₂₆ClN₃O₉S | 580,02 | 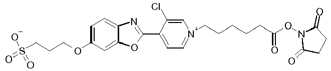 |
| 30 | 5 | C₂₅H₂₅ClN₃O₁₂S₂Na | 682,06 | 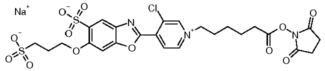 |
| 31 | 6 | C₃₆H₅₀N₃O₁₄Cl | 784,26 | 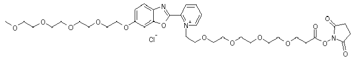 |
| 32 | 7 | C₃₂H₃₅N₄O₁₀Br | 715,56 | 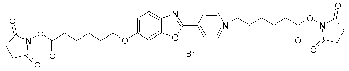 |
| 33 | 8 | C₃₂H₃₄N₄O₁₃S | 714,71 | 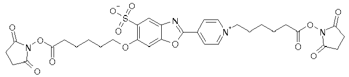 |
| 34 | 9 | C₂₉H₂₉N₃O₉S | 595,63 | 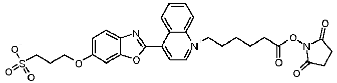 |
| 35 | 10 | C₂₉H₂₈N₃O₁₂S₂Na | 697,68 | 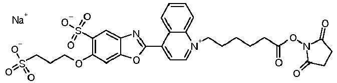 |
| 36 | 11 | C₃₄H₃₉N₃O₁₃S | 729,77 | 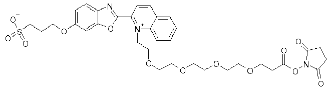 |
| 37 | 12 | C₃₀H₂₅N₃O₈S | 587,61 | 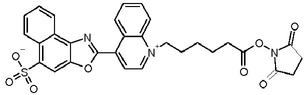 |
| 38 | 13 | C₂₇H₂₉N₃O₉S | 571,61 | 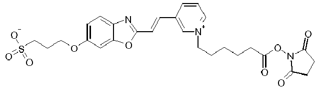 |
| 39 | 14 | C₂₃H₂₄N₃O₆Br | 518,36 | 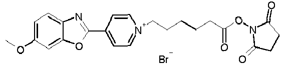 |
| 40 | 15 | C₂₃H₂₃N₃O₉S | 517,52 | 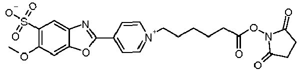 |
| 41 | 17 | C₂₄H₂₄N₄O₉S | 544,54 | 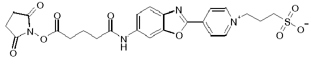 |
| 42 | 18 | C₂₅H₂₇N₄O₇Br | 575,42 | 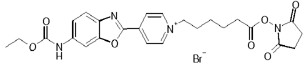 |
| 43 | 19 | C₂₆H₂₉N₅O₉S | 587,61 | 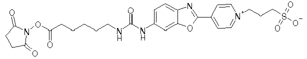 |
| 44 | 20 | C₂₈H₃₃N₄O₁₁S₂Na | 688,71 | 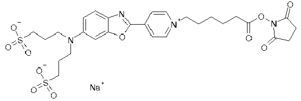 |
| 45 | 21 | C₂₉H₃₄N₅O₁₂S₂Na | 731,74 | 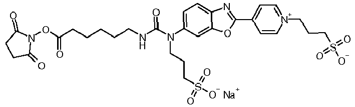 |
| 46 | 22 | C₂₆H₂₂N₃O₁₁S₂Na | 639,60 | 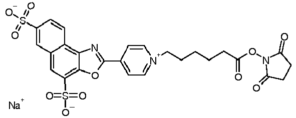 |
| 47 | 23 | C₃₁H₂₆N₃O₁₀S₂Na | 687,68 | 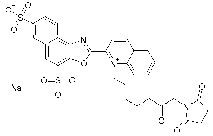 |
| 48 | 24 | C₃₀H₂₄N₃O₁₁S₂Na | 689,66 | 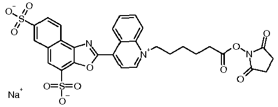 |
| 49 | 25 | C₂₆H₂₂N₃O₁₁S₂Na | 639,60 | 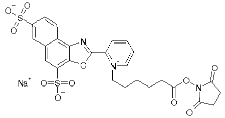 |

### Ausführungsbeispiel 50

### Maleimid der Verbindung 1

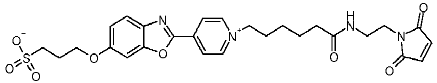

70 mg Verbindung **26** werden in 3 ml DMF gelöst. Bei Raumtemperatur werden 44 µl DIPEA und 36 mg N-(2-Aminoethyl)maleimid-Trifluoroacetat zugegeben und es wird 1 Stunde gerührt. Das Lösungsmittel wird im Vakuum abdestilliert und der Rückstand über eine RP-Säule gereinigt.
Ausbeute: 53 mg (72%) (C₂₇H₃₀N₄O₈S; 570,63 g/mol)
MS ESI + (m/z): 571 (30%, [M + H]⁺); 593 (base, [M + Na]⁺)
UV-Vis in PBS: λₘₐₓ: 383 nm; λₑₘ: 516 nm; ε = 21.500 l/mol*cm

### Ausführungsbeispiel 51

### Amin der Verbindung 25

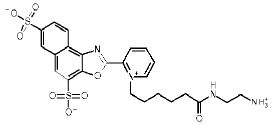

145 mg Ethylendiamin-dihydrochlorid werden in 500 µl Wasser gelöst, mit 95 µl DIPEA versetzt und bei 0°C zu einer Lösung von 70 mg Verbindung 49 in 3 ml DMF gegeben. Diese Reaktionslösung wird 20 Minuten bei 0 °C und 20 Minuten bei Raumtemperatur gerührt. Das Lösungsmittel wird im Vakuum abdestilliert und der Rückstand über eine RP-Säule gereinigt.
Ausbeute: 39 mg (63%) (C₂₄H₂₆N₄O₈S; 562,62 g/mol)
MS ESI - (m/z): 561 (base, [M - H]⁻)
UV-Vis in PBS: λₘₐₓ: 369 nm; λₑₘ: 476 nm; ε = 13.800 l/mol*cm; QY: 0,85

### Ausführungsbeispiel 52

### Titration eines Streptavidin-Konjugats der Verbindung 19 mit dem Biotin-DYQ-1-Quencher-Konjugat (vgl. Fig. 1A und 1B)

Eine 4,7 µM Lösung eines Streptavidin-Konjugats der Verbindung 19 in PBS wurde sukzessive mit einer DYQ-1-Biotin-Lösung (2,5 mM in PBS) versetzt, wobei Stoffmengenverhältnisse von 1:0, 1: 0,5, 1:1, 1:2, 1:4 und 1:6, jeweils auf Streptavidin vs. DYQ-1-Quencher bezogen, eingesetzt wurden. Das spektrale Ergebnis der Titration zeigt Fig. 1A. Um ein falsch-positives Ergebnis durch Reabsorption des Fluoreszenzsignals des Streptavidin-Konjugats durch die zunehmende Menge an DYQ-1-Biotin auszuschließen, wurde als Blindprobe eine gleiche Menge Streptavidin-Konjugat der Verbindung 19 mit gleichen Stoffmengen wie oben des unkonjugierten DYQ-1-Quenchers titriert. Das spektrale Ergebnis ist in Fig. 1B dargestellt.

Es erscheint offensichtlich, dass die Auslöschung der Emission des Streptavidin-Konjugats der Verbindung 19 durch das DYQ-1-Biotin deutlich effizienter erfolgt als im Falle des unkonjugierten Quenchers, was auf einen erfolgreichen Energietransfer zwischen Verbindung 19 als Donor und DYQ-1-Biotin als Akzeptor schließen lässt.

Emissionsspektren der erfindungsgemäßen Verbindungen 1, 17, 19, 21 und 25 sind in der Fig. 7 gezeigt und durch unterschiedliche Stricharten entsprechend der Legende gekennzeichnet.

## Patentansprüche

1. Verbindungen der allgemeinen Struktur mit n = 0 oder 1 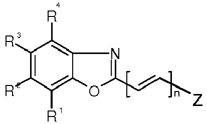 und ihre Salze, wobei Z entweder
Z=4-Pyridyl- 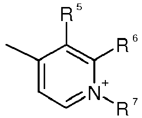 ist und die Struktur I gebildet ist oder
Z = 3-Pyridyl- 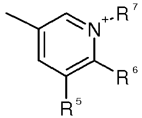 ist und die Struktur II gebildet ist oder
Z = 2-Pyridyl- 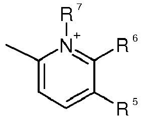 ist und die Struktur III gebildet ist
und R2 und / oder R7 je eine über einen Linker L gebundene reaktive Gruppe A zur kovalenten Bindung von Verbindungen der Strukturen I, II oder III mit einem zu markierenden Molekül K ist, wobei A eine Amin- (-NH₂), Hydroxy- (-OH) oder Phosphoramidit-Funktion (-O-P-[O-CH₂-CH₂-CN]-N[(CH(CH₃)₂]₂), eine Carbonsäure (-COOH), ein davon abgeleiteter Alkyl- oder Aktiv-Ester (NHS-Ester, Sulfo-NHS-Ester, Tetrafluoro-Phenylester, p-Sulfo-Tetrafluoro-Phenylester); ein Carbonsäure-Hydrazid (-CONHNH₂); oder ein Carbonsäure-Amid (-CONHR12)
mit R12 gleich -(CH₂)ₜ-Y, wobei
Y gleich -OH, -NH₂, -NH₃⁺, Maleimid (-N[CO-CH]₂), -NCS, -NCO, -NH-CO-CH₂-I, -NH-CO-CH₂-Br, -Azid (-N3), -Alkin (-CCH) oder-Phosphoramidit (-O-P-[O-CH₂-CH₂-CN]-N-[CH-(CH₃)₂]₂) und
K eine kovalent gebundene Komponente, ausgewählt aus der Gruppe Haptene, Proteine (Antikörper), niedermolekulare Arzneistoffe, Peptide, Nucleotide, Nucleoside, DNA-Oligomere, Polymere ist, und t die Zahlen von 1-10 darstellt,
L gleich ein Linker ausgewählt aus einer Gruppe mit -(CH₂)ₛ-, -[(CH₂)ₘ-O]p-(CH₂)ₘ-, NR10-(CH₂)ₛ-, -O-(CH₂)ₛ-, -NR10-C(O)-(CH₂)ₛ-, -NR10-C(O)-O-(CH₂)ₛ- oder -NR10-C(O)-NR11-(CH₂)ₛ- ist, wobei R10 und R11 unabhängig voneinander Wasserstoff, Alkyl und Alkoxy-Alkyl (-[(CH₂)ₘ-O]ₚ- CH₃), ω-Sulfonsäure-Alkyl (-(CH₂)ᵣ-SO₃), m die Zahlen 2 - 5, p, r und s unabhängig voneinander die Zahlen von 1-10 darstellen,
R1, R3 - R6 unabhängig voneinander gleich Wasserstoff, Chlor, Brom oder ein Sulfonsäure-Rest sind,
im Falle dass R2 LA ist, ist L gleich NR10-(CH₂)ₛ-, -O-(CH₂)ₛ-, NR10-C(O)-(CH₂)ₛ-, -NR10-C(O)-O-(CH₂)ₛ-oder -NR10-C(O)-NR11-(CH₂)ₛ-,
im Falle dass R2 nicht LA ist, ist R2 gleich ω-Sulfonsäure-Alkoxy (-O-(CH₂)ᵣ- SO₃), Alkoxy, Poly-Alkoxy (-O-[(CH₂)ₘ-O]ₚ-CH₃), eine Amin-Funktion (-NR8R9) wobei R8 und R9 unabhängig voneinander Wasserstoff, Alkyl, ω-Sulfonsäure-Alkyl (-(CH₂)ᵣ-SO₃), -CO-Alkyl, -CO-NR10-Alkyl oder -CO-O-Alkyl,
im Falle dass R7 LA ist, ist L gleich -(CH₂)ₛ- oder -[(CH₂)ₘ-O]p-(CH₂)ₘ-,
im Falle dass R7 nicht LA ist, ist R7 gleich ω-Sulfonsäure-Alkyl (-(CH₂)ᵣ-SO₃),
im Falle dass in den Strukuren I und III R3 - R4 gleich -(CR13=CR14)₂- verbrückt sind, wobei R13 und R14 unabhängig voneinander Wasserstoff und /oder Sulfonsäure-Rest ist, sind R1, R2, R5, R6 gleich Wasserstoff oder ein Sulfonsäure-Rest, wobei mindestens einer von R1, R2, R13, R14 ein Sulfonsäure-Rest ist und R7 gleich -LA mit L gleich -(CH₂)ₛ- oder -[(CH₂)ₘ-O]p-(CH₂)ₘ-,
im Falle dass in den Strukuren I und III R5 - R6 -(CR13=CR14)₂- verbrückt sind, wobei R13 und R14 unabhängig voneinander Wasserstoff und / oder Sulfonsäure-Rest ist, sind R1, R3, R4 gleich Wasserstoff, Chlor, Brom oder ein Sulfonsäure-Rest und R2 gleich Sulfonsäure-Alkoxy (-O-(CH₂)ᵣ-SO₃), C1-C18-Alkoxy oder Poly-Alkoxy (-O-[(CH₂)ₘ-O]ₚ-CH₃ und R7 gleich -LA mit L gleich -(CH₂)ₛ- oder -[(CH₂)ₘ-O]ₚ-(CH₂)ₘ-,
im Falle dass in den Strukuren I und III R3 - R4 und R5 - R6 gleich -(CR13=CR14)₂- verbrückt sind, wobei R13 und R14 unabhängig voneinander Wasserstoff und / oder Sulfonsäure-Rest ist, sind R1, R2gleich Wasserstoff oder ein Sulfonsäure-Rest, wobei mindestens einer von R1, R2, R13, R14 ein Sulfonsäure-Rest ist und R7 gleich -LA mit L gleich -(CH₂)₅-oder-[(CH₂)ₘ-O]ₚ-(CH₂)ₘ-.

2. Verbindungen nach Anspruch 1 mit A in Form eines Aktiv-Esters, wobei der Aktiv-Ester gleich ein NHS-Ester (N-Hydroxysuccinimidylester), ein Sulfo-NHS-Ester (Sulfo-Hydroxysuccinimidylester), ein TFP-Ester (Tetrafluoro-Phenylester) oder ein STP-Ester (p-Sulfo-Tetrafluoro-Phenylester) ist.

3. Verbindungen nach Anspruch 1, **dadurch gekennzeichnet, dass** A in Form eines Carbonsäurederivates vorliegt.

4. Verbindungen nach Anspruch 1, **dadurch gekennzeichnet, dass** A in Form eines Phosphoramidits vorliegt, wobei A eine Gruppe der Formel 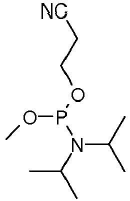 ist.

5. Verwendung der Verbindungen nach einem der Ansprüche 1 bis 4 als Markerfarbstoffe auf der Basis von Benzoxazolen, wobei die zu markierenden Moleküle K über den Linker L und die reaktive Gruppe A an eine der Verbindungen der Strukturen I, II, III gebunden werden.

## Claims

1. Compounds having the general structure where n = 0 or 1 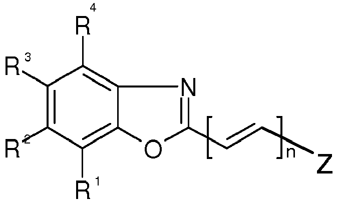 and salts thereof, wherein Z is either
Z = 4-pyridyl 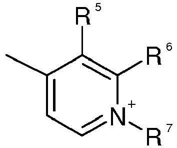 to form structure I, or
Z = 3-pyridyl 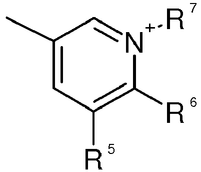 to form structure II, or
Z = 2-pyridyl 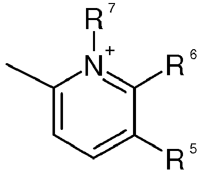 to form structure III,
and R2 and/or R7 are each a reactive group A bound via a linker L for covalently bonding compounds of the structures I, II or III to a molecule K to be marked,
wherein A is an amine (-NH₂), hydroxy (-OH) or phosphoramidite function (-O-P-[O-CH₂-CH₂-CN]-N[(CH(CH₃)₂]₂), a carboxylic acid (-COOH), an alkyl or active ester derived therefrom (NHS ester, sulfo-NHS ester, tetrafluorophenyl ester, p-sulfotetrafluorophenyl ester); a
carboxylic acid hydrazide (-CONHNH₂); or a carboxylic acid amide (-CONHR12)
where R12 is -(CH₂)ₜ-Y, wherein
Y is -OH, -NH₂, -NH₃⁺, maleimide (-N[CO-CH]₂), -NCS, -NCO, -NH-CO-CH₂-I, -NH-CO-CH₂-Br, azide (-N3), alkyne (-CCH) or phosphoramidite (-O-P-[O-CH₂-CH₂-CN]-N-[CH-(CH₃)₂]₂), and
K is a covalently bound component selected from the group haptens, proteins (antibodies), low molecular weight drug compounds, peptides, nucleotides, nucleosides, DNA oligomers, polymers, and t represents the numbers of 1-10,
L is a linker selected from a group featuring -(CH₂)ₛ-, -[(CH₂)ₘ-O]ₚ-(CH₂)ₘ-, -NR10-(CH₂)ₛ-, -O-(CH₂)ₛ-, -NR10-C(O)-(CH₂)ₛ-, -NR10-C(O)-O-(CH₂)ₛ-, or -NR10-C(O)-NR11-(CH₂)ₛ-, wherein R10 and R11 are each independently hydrogen, alkyl and alkoxy-alkyl (-[(CH₂)ₘ-O]ₚ-CH₃), ω-sulfoalkyl (-(CH₂)ᵣ-SO₃), m represents the numbers of 2-5, p, r and s each independently represent the numbers of 1-10,
R1 and R3 - R6 are each independently hydrogen, chlorine, bromine or a sulfonic acid moiety,
in case R2 is LA, L is -NR10-(CH₂)ₛ-, -O-(CH₂)ₛ-, -NR10-C(O)-(CH₂)ₛ-, -NR10-C(O)-O-(CH₂)ₛ- or -NR10-C(O)-NR11-(CH₂)ₛ-,
in case R2 is not LA, R2 is equal to ω-sulfoalkoxy (-O-(CH₂)ᵣ- SO₃), alkoxy, polyalkoxy (-O-[(CH₂)ₘ-O]p-CH₃), an amine function (-NR8R9), wherein R8 and R9 are each independently hydrogen, alkyl, ω-sulfoalkyl (-(CH₂)ᵣ-SO₃), -CO-alkyl, -CO-NR10-alkyl or -CO-O-alkyl,
in case R7 is LA, L is equal to -(CH₂)ₛ- or -[(CH₂)ₘ-O]p-(CH₂)ₘ-,
in case R7 is not LA, R7 is equal to ω-sulfoalkyl (-(CH₂)ᵣ-SO₃),
in case that in the structures I and III R3-R4 are bridged -(CR13=CR14)₂-, wherein R13 and R14 are each independently hydrogen and/or a sulfonic acid moiety, R1, R2, R5 and R6 are each hydrogen or a sulfonic acid moiety, wherein at least one of R1, R2, R13, R14 is a sulfonic acid moiety, and R7 is equal to -LA where L is - (CH₂)ₛ- or -[(CH₂)ₘ-O]p-(CH₂)ₘ-,
in case that in the structures I and III R5-R6 are bridged -(CR13=CR14)₂-, wherein R13 and R14 are each independently hydrogen and/or a sulfonic acid moiety, R1, R3 and R4 are each hydrogen, chlorine, bromine and a sulfonic acid moiety, and R2 is sulfoalkoxy (-O-(CH₂)ᵣ-SO₃), C1-C18-alkoxy or polyalkoxy (-O-[(CH₂)ₘ-O]ₚ-CH₃), and R7 is equal to -LA where L is -(CH₂)ₛ- or -[(CH₂)ₘ-O]ₚ-(CH₂)ₘ-,
in case that in the structures I and III R3-R4 and R5-R6 are bridged -(CR13=CR14)₂-, wherein R13 and R14 are each independently hydrogen and/or a sulfonic acid moiety, R1 and R2 are each hydrogen or a sulfonic acid moiety, wherein at least one of R1, R2, R13, R14 is a sulfonic acid moiety, and R7 is equal to -LA where L is -(CH₂)ₛ- or -[(CH₂)ₘ-O]ₚ-(CH₂)-.

2. Compounds according to claim 1 where A is present in the form of an active ester, wherein the active ester is an NHS ester (N-hydroxysuccinimidyl ester), a sulfo-NHS ester (sulfo-hydroxysuccinimidyl ester), a TFP ester (tetrafluorophenyl ester) or an STP ester (p-sulfotetrafluorophenyl ester).

3. Compounds according to claim 1, **characterized in that** A is present in the form of a carboxylic acid derivative.

4. Compounds according to claim 1, **characterized in that** A is present in the form of a phosphoramidite, wherein A is a group of the formula

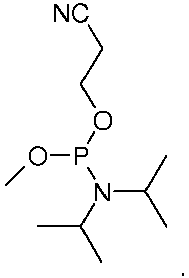

5. Use of the compounds according to one of claims 1 to 4 as benzoxazole-based marker dyes, wherein the molecules K to be marked are bonded to one of the compounds of the structures I, II, III via the linker L and the reactive group A.

## Revendications

1. Composés de la structure générale, dans lesquels n = 0 ou 1, 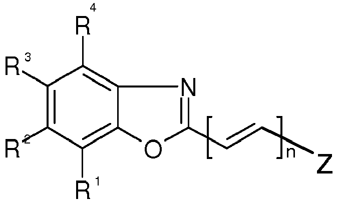 et leurs sels, Z étant soit
Z = 4-pyridyle 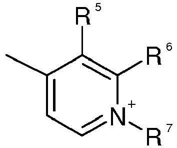 et la structure I étant formée, soit
Z = 3-pyridyle 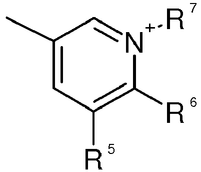 et la structure II étant formée, soit
Z = 2-pyridyle 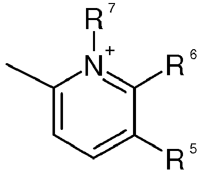 et la structure III étant formée,
et R2 et/ou R7 étant chacun un groupe réactif A lié via un lieur L pour lier de manière covalente des composés des structures I, Il ou III à une molécule K à marquer,
A étant une fonction amine (-NH₂), hydroxy (-OH) ou phosphoramidite (-O-P-[O-CH₂-CH₂-CN]-N[(CH(CH₃)₂]₂), un acide carboxylique (-COOH), un ester d'alkyle ou actif dérivé de ceux-ci (un ester NHS, un ester sulfo-NHS, un ester tetrafluorophenyle, un ester p-sulfotetrafluorophenyle); un
hydracide d'acide carboxylique (-CONHNH₂); ou un amide d'acide carboxylique (-CONHR12),
dans lequel R12 est -(CH₂)ₜ-Y,
Y étant -OH, -NH₂, -NH₃⁺, maléimide (-N[CO-CH]₂), -NCS, -NCO, -NH-CO-CH₂-I, -NH-CO-CH₂-Br, azoture (-N3), alcyne (-CCH) ou phosphoramidite (-O-P-[O-CH₂-CH₂-CN]-N-[CH-(CH₃)₂]₂), et
K étant un composant lié de manière covalente sélectionné parmi le groupe d'haptènes, protéines (anticorps), composés médicamenteux de bas poids moléculaire, peptides, nucléotides, nucléosides, oligomères d'ADN, polymères, et t représentant les nombres de 1-10,
L étant un lieur sélectionné d'un groupe de -(CH₂)ₛ-, -[(CH₂)ₘ-O]ₚ-(CH₂)ₘ-, -NR10-(CH₂)ₛ-, -O-(CH₂)ₛ-, -NR10-C(O)-(CH₂)ₛ-, -NR10-C(O)-O-(CH₂)ₛ- ou -NR10-C(O)-NR11-(CH₂)ₛ-, R10 et R11 chacun indépendamment représentant hydrogène, alkyle et alkoxy-alkyle (-[(CH₂)ₘ-O]ₚ-CH₃), ω-sulfoalkyle (-(CH₂)ᵣ-SO₃), m représentant les nombres de 2-5, p, r et s chacun indépendamment représentant les nombres de 1-10,
R1 et R3 - R6 chacun indépendamment étant hydrogène, chlore, brome ou un reste d'acide sulfonique,
au cas où R2 est LA, L est -NR10-(CH₂)ₛ-, -O-(CH₂)ₛ-, -NR10-C(O)-(CH₂)ₛ-, -NR10-C(O)-O-(CH₂)ₛ- ou -NR10-C(O)-NR11-(CH₂)ₛ-,
au cas où R2 n'est pas LA, R2 est ω-sulfoalkoxy (-O-(CH₂)ᵣ-SO₃), alkoxy, polyalkoxy (-O-[(CH₂)ₘ-O]ₚ-CH₃), une fonction amine (-NR8R9), R8 et R9 chacun indépendamment étant hydrogène, alkyle, ω-sulfoalkyle (-(CH₂)ᵣ-SO₃), -CO-alkyle, -CO-NR10-alkyle ou -CO-O-alkyle,
au cas où R7 est LA, L est -(CH₂)ₛ- ou -[(CH₂)ₘ-O]p-(CH₂)ₘ-,
au cas où R7 n'est pas LA, R7 est ω-sulfoalkyle (-(CH₂)ᵣ-SO₃),
au cas où dans les structures I et III R3-R4 sont pontés -(CR13=CR14)₂-, R13 et R14 chacun indépendamment étant hydrogène et/ou un reste d'acide sulfonique, R1, R2, R5 et R6 chacun sont hydrogène ou un reste d'acide sulfonique, au moins un de R1, R2, R13, R14 étant un reste d'acide sulfonique, et R7 étant -LA, dans lequel L est -(CH₂)ₛ- ou -[(CH₂)ₘ-O]ₚ-(CH₂)ₘ-,
au cas où dans les structures I et III R5-R6 sont pontés -(CR13=CR14)₂-, R13 et R14 chacun indépendamment étant hydrogène et/ou un reste d'acide sulfonique, R1, R3 et R4 chacun sont hydrogène, chlore, brome ou un reste d'acide sulfonique, et R2 est sulfoalkoxy (-O-(CH₂)ᵣ-SO₃), C1-C18-alkoxy ou polyalkoxy (-O-[(CH₂)ₘ-O]ₚ-CH₃), et R7 est -LA, dans lequel L est -(CH₂)ₛ- ou -[(CH₂)ₘ-O]ₚ-(CH₂)ₘ-,
au cas où dans les structures I et III R3-R4 et R5-R6 sont pontés -(CR13=CR14)₂-, R13 et R14 chacun indépendamment étant hydrogène et/ou un reste d'acide sulfonique, R1 et R2 chacun sont hydrogène ou un reste d'acide sulfonique, au moins un de R1, R2, R13, R14 étant un reste d'acide sulfonique, et R7 étant -LA, dans lequel L est -(CH₂)ₛ- ou -[(CH₂)ₘ-O]ₚ-(CH₂)ₘ-.

2. Composés selon la revendication 1, dans lesquels A est présent sous la forme d'un ester actif, l'ester actif étant un ester NHS (ester N-hydroxysuccinimidyle), un ester sulfo-NHS (ester sulfo-hydroxysuccinimidyle), un ester TFP (ester tetrafluorophenyle) ou un ester STP (ester p-sulfotetrafluorophenyle).

3. Composés selon la revendication 1, **caractérisé en ce que** A est présent sous la forme d'un dérivé d'acide carboxylique.

4. Composés selon la revendication 1, **caractérisé en ce que** A est présent sous la forme d'un phosphoramidite, A étant un groupe de la formule

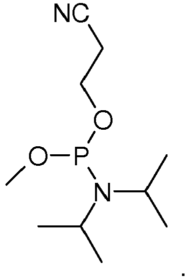

5. Utilisation des composés selon une des revendications 1 à 4 en tant que colorants de marqueur à base de benzoxazoles, les molécules K à marquer étant liées à un des composés des structures I, II, III via le lieur L et le groupe réactif A.
